# FASCICULE DE BREVET EUROPEEN

(11) **EP 1 373 563 B1**
(45) Date de publication et mention de la délivrance du brevet: **29.03.2006**
(21) Numéro de dépôt: 01956623.1
(22) Date de dépôt: 20.07.2001
(51) Int. Cl.: C12Q 1/68

(54) **PROCEDE DE DETECTION DE MICROORGANISMES**
VERFAHREN ZUM NACHWEIS VON MIKROORGANISMEN
METHOD FOR DETECTING MICRO-ORGANISMS

(30) Priorité: 21.07.2000 FR 0009600; 02.10.2000 FR 0012524
(43) Date de publication de la demande: 02.01.2004
(73) Titulaire: Université d'Auvergne, 63000 Clermont-Ferrand (FR)
(72) Inventeur: ROMOND, Pierre-Charles, F-63670 Orcet (FR); RENAUD, Michel, F-63670 Le Cendre (FR); ALRIC, Monique, F-63000 Clermont-Ferrand (FR); MEINIEL, Olivier, F-63800 Cournon d'Auvergne (FR); BALLUT, Lionel, F-63400 Chamalière (FR)
(74) Mandataire: Warcoin, Jacques
(86) Numéro de dépôt international: PCT/FR2001/002371
(87) Numéro de publication internationale: WO 2002/008459

(56) Documents cités:
- EP-A- 0 472 434
- WO-A-97/29212
- FR-A- 2 752 425
- US-A- 5 536 638
- US-A- 5 756 293
- US-A- 5 786 147
- US-A- 5 989 821
- HARADA HOSAMI ET AL: "Phylogenetical relationship based on groE genes among phenotypically related Enterobacter, Pantoea, Klebsiella, Serratia and Erwinia species." JOURNAL OF GENERAL AND APPLIED MICROBIOLOGY, vol. 43, no. 6, décembre 1997 (1997-12), pages 355-361, XP008011235 ISSN: 0022-1260
- ZAKHAROVA NATALYA ET AL: "Fused and overlapping rpoB and rpoC genes in Helicobacters, Campylobacters, and related bacteria." JOURNAL OF BACTERIOLOGY, vol. 181, no. 12, juin 1999 (1999-06), pages 3857-3859, XP002222693 ISSN: 0021-9193
- RENESTO PATRICIA ET AL: "rpoB gene analysis as a novel strategy for identification of spirochetes from the genera Borrelia, Treponema, and Leptospira" JOURNAL OF CLINICAL MICROBIOLOGY, WASHINGTON, DC, US, vol. 38, no. 6, juin 2000 (2000-06), pages 2200-2203, XP002155811 ISSN: 0095-1137
- LOVE B C ET AL: "Cloning and sequence of the groESL heat-shock operon of Pasteurella multocida" GENE, ELSEVIER BIOMEDICAL PRESS. AMSTERDAM, NL, vol. 166, no. 1, 1995, pages 179-180, XP004043133 ISSN: 0378-1119
- OHTA T ET AL: "MOLECULAR CHARACTERIZATION OF THE GENE OPERON OF HEAT SHOCK PROTEINS HSP60 AND HSP10 IN METHICILLIN-RESISTANT STAPHYLOCOCCUS AUREUS" BIOCHEMICAL AND BIOPHYSICAL RESEARCH COMMUNICATIONS,US,ACADEMIC PRESS INC. ORLANDO, FL, vol. 193, no. 2, 15 juin 1993 (1993-06-15), pages 730-737, XP002018804 ISSN: 0006-291X
- EMELYANOV, V. V. ET AL: "A groE-based phylogenetic analysis shows very close evolutionary relationship between mitochondria and Rickettsia" RUSS. J. GENET. (1999), 35(6), 618-627, XP000993409
- SGHIR ABDELGHANI ET AL: "Quantification of bacterial groups within human fecal flora by oligonucleotide probe hybridization." APPLIED AND ENVIRONMENTAL MICROBIOLOGY, vol. 66, no. 5, mai 2000 (2000-05), pages 2263-2266, XP002166836 ISSN: 0099-2240
- MOLLET C ET AL: "RPOB SEQUENCE ANALYSIS AS A NOVEL BASIS FOR BACTERIAL IDENTIFICATION" MOLECULAR MICROBIOLOGY, BLACKWELL SCIENTIFIC, OXFORD, GB, vol. 26, no. 5, 1997, pages 1005-1011, XP000913977 ISSN: 0950-382X

## Description

La présente invention concerne un procédé de détection des éléments constitutifs d'une flore bacterienne, dont au moins une partie des éléments possède un opéron en commun, caractérisé en ce que l'on identifié les éléments de ladite flore par l'étude de la séquence intergénique dudit opéron.

Le système digestif humain, héberge un nombre considérable de micro-organismes qui constituent une flore microbienne d'une extrême complexité. Bien que ces bactéries soient réparties tout au long du tractus digestif, le côlon renferme l'essentiel de cette flore tant d'un point de vue quantitatif que qualitatif On estime que la flore colique d'un individu est constituée de 10¹³ à 10¹⁵ bactéries, pour l'essentiel anaérobics, représentées par au moins 400 espèces appartenant à environ 30 genres différents. Ces bactéries colonisent les différents étages du côlon de façon relativement hétérogène. On décrit classiquement une flore dite de fermentation au niveau du caccum ainsi qu'une flore dite de putréfaction au niveau du côlon gauche. Par ailleurs, on distingue une flore résidente d'une flore de passage. Cette flore résidente est elle-même scindée en flore dominante et flore sous-dominante. Les bactéries dominantes, essentiellement anaérobies, sont majoritairement représentées par le genre *Bacteroïdes,* bacilles gram négatif, mais aussi par les genres *Bifidobacterium, Lactobacillus* ou *Clostridium,* bacilles gram positif. La flore sous-dominante renferme des bactéries aéro-anaérobies et microaérophiles. On note surtout la présence d'entérobactéries et de streptocoques. Aussi bien le régime alimentaire, les infections, l'apport de pré et probiotiques que les traitements antibiotiques, sont susceptibles de provoquer des modifications drastiques de la composition de la flore colique. Ces variations ayant un impact direct sur la santé, il est important de les connaître et de les comprendre, tant pour les éviter que pour les déclencher dans un but thérapeutique. Jusqu'à présent, l'étude des bactéries coliques a permis de caractériser environ 200 espèces. Toutefois, ce type d'investigation se heurte à de nombreux problèmes liés essentiellement à la lourdeur des techniques. Les résultats obtenus ainsi que les conclusions qui en decouleat sont encore fragmentaires.

L'identification des bactéries est réalisée selon différents procédas qui font appel soit à la mise en évidence de caractères phénotypiques ou biochimiques spécifiques, soit à l'utilisation et la reconnaissance de zones spécifiques hétérologues présentes sur le génome.

Un début d'identification pourra être effectué en décrivant la morphologie de l'organisme étudié et en recherchant par exemple la présence d'endospores, de gaines, de kystes, de bourgeons, de corps fructifiants... La forme des colonies, la pigmentation, l'origine du prélèvement sont également des informations utiles. On pourra également effectuer des études préliminaires en utilisant des colorants spécifiques de la capsule, des flagelles, des granules, de la paroi... Toutefois, une identification plus poussée et précise passe obligatoirement par des techniques d'isolement sur milieux sélectifs. De tels milieux sont développés ou améliorés afin d'augmenter la spécificité de cette sélection. Cependant, il est difficile d'exclure complètement la présence d'éventuels contaminants qui peuvent alors interférer dans les tests de reconnaissance ultérieurement employés.

Ces tests font appel aux caractères biochimiques spécifiques d'une espèce. Il existe des systèmes multitests. Ce sont des galeries d'identification qui se présentent sous forme de kits, de microplaques, de bandelettes dont l'utilisation est parfois automatisée. Toutefois, il existe un certain degré d'hétérogénéité d'origine chromosomique ou plasmidique au sein de nombreuses espèces. Ainsi un ou plusieurs caractères seront absents lors de l'identification. Aussi ne donnera-t-on, la plupart du temps, que la probabilité d'appartenance à une espèce. Une similitude de 80% ou plus avec une bactérie de référence sera considérée comme acceptable. Des systèmes monotests sont également développés. Ils utilisent des substrats synthétiques fluorescents permettant de mettre en évidence la présence d'une enzyme spécifique d'un microorganisme. Ils permettent une analyse rapide mais sont limités dans leur utilisation. En effet, un test spécifique doit être développé pour chaque espèce.

Des tests immunologiques à partir d'anticorps poly- ou monoclonaux sont également développés. En dehors' de la limitation évidente inhérente aux anticorps polyclonaux, ces tests sont majoritairement employés pour la caractérisation de sérotypes mais rarement pour l'identification d'espèces. Bien que couramment utilisés dans le diagnostic hospitalier, ils restent peu employés en bactériologie. Quant à la réalisation d'anticorps monoclonaux, elle reste un travail long, laborieux et onéreux. Ils sont par exemple dirigés contre des lipopolysaccharides, la membrane, les pili..., mais sont cependant rarement spécifiques d'une espèce.

Le développement des techniques de biologie moléculaire a permis de développer de nouveaux tests d'identification. Ces techniques sont basées sur des réactions d'hybridation ou d'amplification en chaîne par polymérase (PCR).

L'hybridation génome/génome doit être supérieure ou égale à 70% pour identifier une espèce de bactérie inconnue comme étant la bactérie de référence connue dont on utilise l'ADN génomique pour réaliser le diagnostic. D'autres tests font intervenir des zones hétérologues sur l'ADN spécifique d'une espèce. On peut ainsi utiliser la technique d'hybridation qui consiste à déposer sur une membrane de nylon ou nitrocellulose le produit à analyser puis à incuber avec une sonde marquée (sonde froide ou sonde chaude) spécifique {1}.

On peut également employer des amorces spécifiques permettant d'amplifier un fragment de taille déterminée par la technique de PCR {2, 3}. Dans ce cas, les amplifiats obtenus par PCR peuvent être eux-mêmes analysés par d'autres techniques telles que la RFLP (polymorphisme de taille des fragments de restriction) {4} ou le TGGE (gel d'électrophorèse en gradient de température) {5}, affinant le diagnostic.

En dépit de leur grande capacité de discrimination, ces techniques restent limitées dans la mesure ou elles ne permettent d'analyser qu'une espèce à la fois, ou bien consiste à isoler un mélange d'espèces qu'on ne peut identifier alors sans connaître exactement le pattern d'analyse des amplifiats par une technique donnée sur un biotope donné.

Le développement des puces à ADN (biopuces) permet d'envisager un diagnostic rapide portant sur plusieurs centaines d'espèces. Cette technique consiste à placer sur une surface de quelques millimètres carrés plusieurs centaines de séquences d'ADN spécifiques d'un organisme donné. Ces sondes sont hybridées avec des fragments d'ADN, généralement obtenus par RT-PCR. L'hybridation éventuelle desdits fragments est alors observée, et indique la présence ou l'absence du gène exprimé, ou de l'organisme étudié.

Les cibles nucléiques étudiées ces dernières années sont essentiellement l'ARN ribosomal 16S (avec plus de 7000 séquences disponibles) {1, 2, 4, 5}, la région séparant les loci génétiques 16S et 23S {3} et les facteurs d'élongation {6, 7}. Ainsi, en se basant sur les ARN 16S, plusieurs espèces nouvelles ont pu être détectées, appartenant aux groupes *Bactéroïdes* et *Clostridium,* mais pas *Bifidobacterium* {8}. Par ailleurs, et bien que les ARN 16S permettent de détecter et d'identifier de nombreuses bactéries au degré de l'espèce, ils sont incapables de discriminer les différentes espèces de Staphylocoques {2, 3}. Ainsi, une région variable du gène codant pour HSP 60 a été proposée pour l'étude des microorganismes de la flore intestinale {9}.

On connaît aussi {11} que l'amplification d'une séquence nucléotidique variable du gène codant pour la protéine HSP60 conservée, à l'aide d'amorces dessinées dans les régions flanquantes très conservées, permet la détection de bactéries, en particulier de staphylocoques, au niveau de l'espèces.

D'autre part, on sait également {12} détecter la présence des trois genres de Spirochètes par amplification PCR du gène codant la sous-unité β de l'ARN polymérase (gène rpoB) à l'aide d'amorces dessinées dans les séquences conservées. L'amplification du gène codant rpoB ne permet toutefois pas d'amplifier des séquences de bactéries appartenant à d'autres genres, telles que *Escheria coli* et *Staphylococcus aureus.*

La présente invention a pour objet un procédé de détection et d'identification des éléments constitutifs d'une flore bactérienne, en particulier la flore intestinale, selon lequel on détecte et en étudie une cible encore plus discriminatoire et universelle que celles déjà étudiées.

Le procédé selon la présente invention comprend la caractérisation des séquences de cette cible pour les organismes présents dans la flore bactérienne étudiée, et permet ainsi de concevoir un test de diagnostic.

Ainsi, la cible étudiée dans le procédé de l'invention présente une forte hétérogénéité interespèce, ce qui permet la discrimination entre les microorganismes,

La présente invention concerne donc un procédé de détection des éléments constitutifs d'une flore bactérienne dont au moins une partie des éléments possède un opéron en commun, caractérisé en ce que:
a) on prépare l'ADN génomique de ladite flore ou les ARNm,
b) on amplifie au moins une partie des séquences intergéniques non codantes situées dans l'opéron conservé chez au moins une partie des éléments de la flore et
c) on identifie les différentes séquences intergéniques amplifiées afin de déterminer les éléments de ladite flore,
ledit opéron étant un opéron rpoBC.

En effet, de façon surprenante, il a été remarqué que les régions intergéniques, dans l'opéron rpoBC conservé entre différentes espèces, présentent une certaine hétérogénéité, alors que les zones codantes qui flanquent lesdites régions en 5' et/ou en 3' sont généralement très conservées. Il est possible que ce fait soit du à une pression de sélection peu forte sur les régions non codantes au cours de l'évolution {10}.

L'amplification est effectuée de préférence par amplification en chaîne par polymérase (PCR), mais d'autres méthodes (PCR-like) peuvent être employées, utilisant un couple d'amorces de séquences nucléotidiques permettant la mise en oeuvre du procédé selon l'invention.

Par PCR-like on entend désigner toutes les méthodes mettant en oeuvre des reproductions directes ou indirectes des séquences d'acides nucléiques, ou bien dans lesquelles les systèmes de marquage ont été amplifiés, ces techniques sont bien entendu connues. En général il s'agit de l'amplification de l'ADN par une polymérase ; lorsque l'échantillon d'origine est un ARN il convient préalablement d'effectuer une transcription reverse. Il existe actuellement de très nombreux procédés permettant cette amplification, comme par exemple la technique SDA. (Strand Displacement Amplification) ou technique d'amplification à déplacement de brin, la technique TAS (Transcription-based Amplification System), la technique 3SR (Self-Sustained Séquence Replication), la technique NASBA (Nucleic Acid Séquence Based Amplification), la technique TMA (Transcription Mediated Amplification), la technique LCR (Ligase Chain Reaction), la technique de RCR (Repair Chain Reaction), la technique CPR (Cycling Probe Reaction), la technique d'amplification à la Q-béta-réplicase. Certaines de ces techniques ont depuis été perfectionnées.

L'analyse des séquences amplifiées est avantageusement effectuée sur une puce à ADN comportant des séquences complémentaires des séquences susceptibles d'être amplifiées à partir des éléments de ladite flore. La connaissance des microorganismes pouvant être présents dans l'échantillon biologique étudié est donc importante afin de choisir la puce à ADN à utiliser. Ainsi, il est nécessaire que la puce à ADN présente, à sa surface, des sondes spécifiques de chacua des organismes que l'on veut étudier, Une telle puce à ADN est également un objet de l'invention.

Ainsi, la présente invention concerne tout particulièrement une puce à ADN comprenant, à sa surface des séquences complémentaires aux séquences intergéniques non codantes situées dans un opéron conservé entre différentes espèces, ledit opéron étant un opéron rpoBC. Par puce à ADN, on entend un support solide sur lequel on fixe des fragments d'acides nucléiques dans des conditions permettant leur hybridation avec les oligonucléotides complémentaires et la détection des hybrides ainsi formés. Ainsi, une puce à ADN selon l'invention concerne également les membranes telles qu'utilisées pour effectuer des Southem Blots.

Les oligonucléotides fixés sur la puce à ADN selon la présente invention le sont par toute méthode classique connue de l'homme du métier, et présentent une longueur d'environ 50 bases. Il est entendu que les oligonucléotides considérés peuvent également être plus courts, ou plus long. Ainsi, il est à la portée de l'homme du métier de déterminer la longueur des oligonucléotides fixés sur la puce selon l'invention, pour chaque séquence.

De façon préférée, les oligonucléotides fixés sur la puce à ADN sont choisis de telle sorte que leur séquence comprenne une partie de la région hypervariable identifiée selon la présente invention. Les oligonucléotides fixés sur la puce selon l'invention peuvent également contenir des séquences correspondant aux séquences variables à un degré moindre, situées à ou proche de l'extrémité des gènes de l'opéron.

Dans un cas particulier et préféré de mise en oeuvre de l'invention, la puce à ADN selon l'invention présente une pluralité (un nombre supérieur ou égal à 2, de préférence 3, de façon plus préférée 5, de façon la plus préférée 10) d'oligonucléotides de taille supérieure à 30 bases. De préférence, lesdits oligonucléotides comprennent un fragment d'au moins 40 ou 50, de façon plus préférée 75, de façon la plus préférée 100 bases consécutives, des séquences SEQ ID N° 63 à SEQ ID N° 138, correspondant aux séquences intergéniques de différentes espèces (rpoBC pour SEQ ID N°63 à SEQ ID N°138).

Ainsi, la mise en évidence des éventuelles hybridations des séquences amplifiées permet d'identifier les éléments présents dans la flore microbienne étudiée.

L'opéron adapté à la mise en oeuvre du procédé selon l'invention est l'opéron rpoBC des bactéries. Cet opéron bactérien contient des séquences codantes relativement homologues entre genres. Il est donc possible de déterminer des amorces dégénérées pour amplifier une zone hétérologue entre espèces qui correspond à la zone intergénique transcrite (ZIG). L'opéron rpoBC code chez les bactéries pour les sous-unités beta et beta prime de la « DNA-directed RNA polymerase» (ARN polymérase) tout comme les gènes homologues conservés ou non sous forme d'opéron chez les mitochondries et autres organelles eucaryotiques (chloroplastes), et tout comme l'ARN polymérase II eucaryote nucléaire (qui synthétise les ARN messagers). L'étude de cet opéron permet non seulement de détecter les bactéries mais aussi d'autres microorganismes encaryotes (levures, protozoaires, ou autres).

Le procédé selon l'invention est ainsi mis en oeuvre, en utilisant des amorces dégénérées situées dans les séquences codantes de l'opéron rpoBC notamment au moins une amorce choisie parmi les séquences SEQ ID N° 1 à SEQ ID N° 31, et SEQ ID n°5 à SEQ ID n°6, elles-mêmes objet de l'invention. Les protéines ARN polymérases sont en effet extrêmement conservées selon les espèces, ce qui permet de trouver des séquences d'acides aminés s'alignant entre elles, et ainsi de choisir des oligonucléotides dégénérés pour effectuer l'amplification des séquences intergéniques.

On utilise les couples d'amorces décrites par les séquences, (une séquence choisie parmi les séquences SEQ ID N° 1 à SEQ ID N° 8) / (une séquence choisie parmi les séquences SEQ ID N° 9 à SEQ ID N° 11) pour amplifier une première fois la région intergénique ZIG des bacteries. Une seconde amplification plus spécifique peut alors être mise en oeuvre en utilisant des couples d'amorces s'hybridant à l'intérieur de la première région amplifiée et décrites par les séquences, (une séquence choisie parmi les séquences SBQ ID N° 12 à SEQ ID N° 15) / (une séquence choisie parmi les séquences SEQ ID N° 16 à SBQ ID N° 31).

On utilise les couples d'amorces décrites par les séquences, une séquence choisie parmi les séquences SEQ ID N° 53 et SEQ ID N° 54 pour amplifier la région intergénique ZIG des bacteries.

Ces amorces ont été dessinées à partir de l'étude de la dégénérescence de motifs protéiques conservés correspondant à rpoB et/ou codés par le gène rpoB : Pour les séquences inverses, déterminées à partir de la dégébérescence de motifs protéiques conservés correspondant à rpoC et/ou codés par le gène rpoC beta p 2 i :

Ces amorces font aussi partie de l'invention.

L'invention a également pour objet les séquences génomiques de microorganismes pouvant être amplifiées par les amorces selon l'invention, en particulier les couples d'amorces (une séquence choisie parmi les séquences SEQ ID N° 1 à SEQ ID N° 8) / (une séquence choisie parmi les séquences SEQ ID N° 9 à SEQ ID N° 11) et les couples d'amorces (une séquence choisie parmi les séquences SEQ ID N° 12 à SEQ ID N° 15) / (une séquence choisie parmi les séquences SEQ ID N° 16 à SEQ ID N° 31). On envisage aussi l'amplification avec des couples d'amorces (une séquence choisie parmi les séquences SEQ ID N° 53, SEQ ID N° 55 à SEQ ID N° 58) / (une séquence choisie parmi les séquences SEQ ID N° 54, SEQ ID N° 59 à SEQ ID N° 61).

Ainsi, l'invention a également pour objet une séquence parmi SEQ ID N° 63 à SEQ ID N° 138, qui correspondent aux régions intergéniques hypervariables de l'opéron xpoB de différents organismes. L'invention a également pour objet tout fragment d'un minimum de 30 bases, de façon plus préférée 50 bases, de façon encore plus préférée 75 bases, de façon la plus préférée 100 bases de l'une des séquences SEQ ID N° 63 à SEQ ID N° 138 ou leurs séquences complémentaires, ledit fragment pouvant être utilisé pour définir des amorces spécifiques des organismes, ou pour l'identification d'organismes notamment par hybridation.

Ainsi, la puce à ADN selon l'invention présente de préférence à sa surface une pluralité d'oligonucléotides (au minimum deux) comprenant des fragments choisis parmi les fragments des séquences SEQ ID N° 63 à SEQ ID N° 138 définis ci-dessus, permettant ainsi l'identification de microorganismes. La taille de ces oligonucléotides peut être déterminée par l'homme du métier, en fonction des conditions d'hybridation qu'il compte mettre en oeuvre. On envisage ainsi des oligonucléotides d'une taille d'environ 50 bases.

La présente invention a également pour objets des trousses de diagnostic pour la mise en oeuvre du procédé selon l'invention. Ces trousses de diagnostic contiennent des amorces dégénérées permettant l'amplification d'une ou plusieurs régions intergéniques de l'opéron rpoBC conservé parmi les espèces. Elles peuvent également contenir les réactifs nécessaires à la réaction d'amplification. Par ailleurs, de l'ADN représentant des contrôles positifs ou négatifs peut être inclus dans les trousses de diagnostic selon l'invention.

Une trousse de diagnostic selon l'invention contient aussi avantageusement les éléments nécessaires pour l'analyse des produits amplifiés. En particulier, une trousse à diagnostic selon l'invention contient une puce à ADN selon l'invention, qui présente, à sa surface, les séquences correspondant aux différents microorganismes.

Selon l'espèce que l'on désire détecter, la trousse de diagnostic selon l'invention contient le couple d'amorces et les éléments d'analyse appropriés. Par ailleurs, une trousse selon l'invention peut également comprendre des instructions pour la mise en oeuvre du procédé selon l'invention.

Une trousse à diagnostic selon l'invention peut aussi ne contenir qu'une puce à ADN selon l'invention, ainsi éventuellement que des instructions permettant la mise en oeuvre de l'analyse de fragments situés dans la région intergénique de l'opéron rpoBC conservé entre les espèces.

Le couplage d'amorces et de sondes spécifiques permet ainsi une identification rapide et précise de la flore d'un sujet donné. On peut donc établir le ou les profils de populations caractéristiques de sujets sains. On peut aussi établir les profils types de différentes pathologies.

Le procédé selon l'invention offre également la possibilité d'un suivi facile de l'évotudon de la flore en fonction de l'alimentation. Plus spécifiquement, on peut suivre les effets, au niveau colique, d'un aliment particulier tel qu'un pré ou probiotique ou d'un traitement médicamenteux tel qu'une antibiothérapie.

On peut donc envisager la mise au point d'aliments ou médicaments à visée « effet sur la flore » permettant un rétablissement et un retour à un profil normal après un déséquilibre faisant suite à une pathologie ou une agression quelconque. On peut également utiliser des amorces et des sondes correspondant à des souches pathogènes afin d'établir éventuellement des seuils critiques de populations précédant une pathologie. On peut alors déterminer quelles sont les autres populations susceptibles d'exercer un effet barrière sur ces pathogènes.

Les outils de diagnostic de la flore intestinale développés et basés sur le procédé selon l'invention (qui sont également objets de l'invention) intéressent dans un premier temps les industriels de l'agro-alimentaire et de la pharmacie afin de mettre au point leurs produits et de voir l'impact de ceux-ci sur la flore intestinale. En effet, des régimes particuliers sont susceptibles à long terme de modifier de façon importante la composition de la flore et d'avoir par conséquent des effets néfastes ou bénéfiques selon les types de populations qui apparaissent ou disparaissent. De la même façon, des traitement médicamenteux et en particulier les traitements antibiotiques entraînent des déséquilibres au sein de la microflore. La caractérisation des populations touchées selon le type de médicament permettrait de mettre en place un traitement parallèle ou ultérieure capable d'empêcher ces modifications ou de rétablir une flore correcte le plus rapidement possible.

Ces outils intéressent aussi les professionnels de la santé pour la caractérisation de la flore intestinale de patients, ce qui peut permettre, par exemple, d'orienter un traitement. En effet, les gastro-entérologues estiment à 70% la part de la population des pays industrialisés se plaignant de troubles digestifs divers que l'on appelle colopathies fonctionnelles celles-ci allant de simples désordres digestifs comme les ballonnements, les flatulences à des désordres plus importants comme la constipation, ou la diarrhée.... La majeure partie de leurs consultations concerne ces colopathies fonctionnelles.

Peu de solutions sont apportées pour soigner ces troubles car pour certaines colopathies leur cause est encore assez méconnue et pour d'autres on ne dispose pas de traitement efficace. A cela s'ajoute le problème du diagnostic médical car les patients présentant ces symptômes de colopathies fonctionnelles ne présentent généralement aucune lésion physique au niveau du côlon. Seul un questionnaire permet au gastro-entérologue de se diriger vers un type de traitement qui se révèle peu efficace dans une majorité des cas. Le marché des produits pouvant soulager ces troubles est donc important, tout comme celui du diagnostic. En effet, un diagnostic de l'état de la flore des patients pourrait renseigner le médecin sur les causes de leurs troubles et le traitement à conduire.

Pour sélectionner une cible génomique d'intérêt, soit une cible qui soit conservée dans tous les génomes, la conservation des opérons les plus conservés au cours de l'évolution a été étudiée à partir des génomes des 51 bactéries entièrement séquencées et disponibles sur le serveur NCBI. Ces séquences ont été positionnées par rapport à celle de rpo B/C (opéron beta).

Il est ressorti de cette première analyse que les cibles les plus longues et les plus conservées sont en effet les opérons groESL (codant pour Hsp10 (groES) et Ilsp60 (groEL)) et une partie de l'operon beta correspondant aux gènes rpoB et rpoC (codant pour les sous-unitées beta et beta' de la DNA-directed-RNA polymerase). De plus, il a été possible d'identifier des motifs protéiques conservés suffisamment longs pour permettre la définition puis la synthèse d'amorces dégénérées universelles (ubiquitaires) où presque.

Ainsi, l'opérons rpoBC a été choisi afin d'exemplifier le principe de la méthode selon l'invention.

Enfin, la zone d'intérêt de l'opéron bêta a été amplifiée, c'est-à-dire la région amplifiable par PCR en utilisant les deux amorces dégénérées correspondantes (FO et RP : SEQ ID N° 53 et SEQ ID N° 54) pour une sélection de bactéries afin d'en établir la séquence et de les tester par hybridation sur membrane de nylon pour valider leur spécificité. Ces séquences ont également été alignées à leurs homologues disponibles sur GenBank afin d'observer cette spécificité par bioinformatique.

Les exemples suivants sont destinés à illustrer l'invention, et ne doivent pas être considérés comme limitant l'invention.
Dans la demande, les abréviations pour les bactéries sont comme suit : Bacillus subtilis (BS) CIP 52-65T; Bacteroides vulgatus (BV) DSM 1447 ; Bilidobacterium longum (BL) DSM 20219 ; Clostridium leptum (CL) DSM 753 ; Clostridium nexile (CN) DSM 1787 ; Clostridium spiroforme (CS) DSM 1552 ; Clostridium glycolycum (CG) DSM 1288 ; Lactobacillus gaseri (LG) DSM 20077 ; Lactobacillus helveticus (LH) CIP 103146; Lactobacillus paracasei (LP) DSM 8741 ; Lactobacillus reuteri (LR) DSM 20053; Pseudomonas neruginosa (PA) CIP100720 ; Ruminococcus hydrogenotrophicus (RH) DSM 10507 ; Citrobacter freundii (CF) ; Serratia liquefaciens (SL) ; Serralia marcescens (SM) ; Enterobacter cloacae (EnC) ; Escherichia coli (EsC) ; Morganella morganii (MM) ; Proteus mirabilis (PM) ; Klebsiella oxytoca (KO) ; Klebsiella pneumoniae (KP)

### DESCRIPTION DES FIGURES

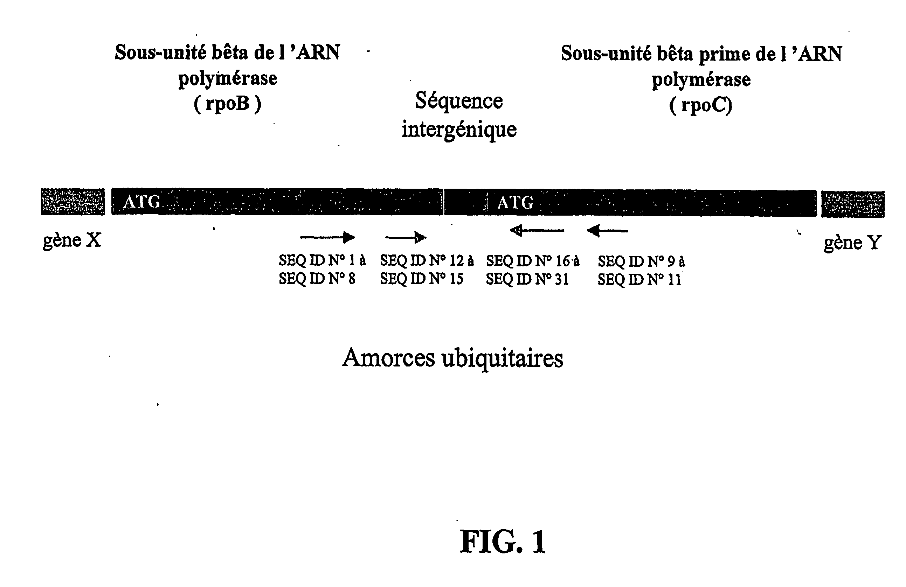

Figure 1: Schéma de l'opéron rpoBC de *E. coli.* Les amorces universelles (ubiquitaires) sont utilisées pour l'amplification de la séquence intergénique.

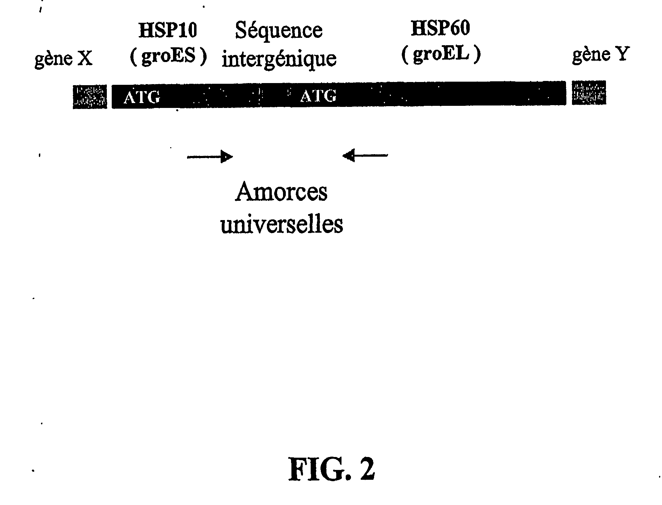

Figure 2: Schéma de l'opéron groESL de *E. coli.* Les amorces universelles sont utilisées pour l'amplification de la séquence intergénique.

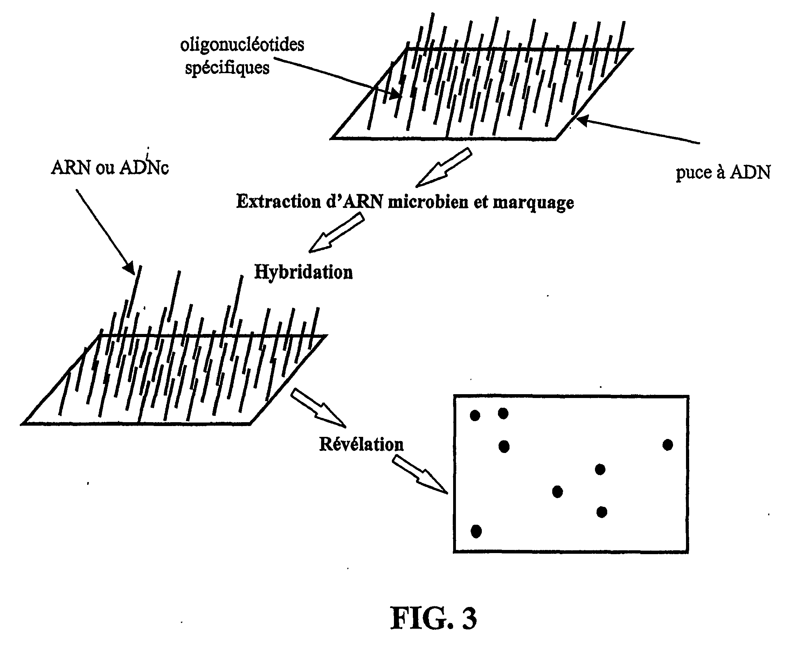

Figure 3 : Principe d'une puce à ADN. Des séquences spécifiques sont fixées sur un support solide. L'hybridation éventuelle des séquences complémentaires permet de déterminer leur présence dans un échantillon.
Figure 4: Hybridation de dépôts de 10 ng d'ADN amplifié par PCR avec des amorces rpoBC (i) et d'ADN génomique (ii) avec une sonde Serratia marcescens, (~0.25 ng / ml (A) ou Klebsiella oxytoca, ~1 ng / ml (B) pendant 18 heures à 60°C et révélation 30 min à 37°C. On peut observer une hybridation croisée de CF, SM, SL, EC et KP avec la sonde KO-DIG (~1 ng / ml)et de SL avec la sonde SM-DIG (~0.25 ng / ml).
Figure 5 : Hybridation de dépôts i (ADN génomique, a:10 à 20 µg, b:5 à 10 µg, c: 0.5 à 1 µg, d: 50 à 100 ng, e: 5 à 10 ng, f: 0.5 à lng) et ii (ADN amplifié par PCR avec des amorces GroESL. a:50 à 100 ng, b:5 à 10 ng, c: 0.5 à 1 ng, d: 50 à 100 pg, e: 5 à 10 pg, f: 0.5 à 1 pg) avec une sonde PA-DIG (~10ng / ml) pendant 18 heures à 42°C et révélation 30 min à 37°C.
Figure 6: Hybridation de dépots i (ADN amplifié par PCR avec des amorces rpoBC : 10ng / 1ng / 100 pg) et ii (ADN génomique: 1µg / 100ng / 10ng) avec une sonde LR-DIG (~1ng / ml) pendant 18 heures à 50, 55, 60 et 65°C et révélation 30 min à 37°C.

### EXEMPLES

### Exemple 1 : Isolement de souches

Afin de posséder un échantillon large et représentatif de la flore colique humaine, il est nécessaire d'isoler de nouvelles souches bactériennes, encore dites non cultivables (et donc inconnues), qui constituent un fort pourcentage des bactéries de la flore colique humaine.

Pour réaliser ces isolements, une grande quantité de selles humaines est collectée et stérilisée au moyen de rayonnements de type gamma ou par la chaleur, en vue d'y ensemencer des échantillons de ces mêmes selles humaines et de les cultiver en aérobiose et anaérobiose, en milieux liquide et solide.

En fonction des conditions de culture utilisées, on peut ainsi isoler de nouveaux genres, espèces ou souches de bactéries coliques ou autres micro-organismes eucaryotes.

### Exemple 2 : Caractérisation des séquences des souches isolées (rpoB)

Il s'agit d'effectuer la caractérisation moléculaire de séquences, idéalement d'ARNm pour effectuer une quantification et sinon d'ADN génomique, des isolats de micro-organismes bactériens ou eucaryotes.

On effectue l'étude des séquences correspondant à des portions de l'opéron bactérien rpoBC. Les gènes de cet opéron sont en effet relativement homologues entre genres.

Une analyse informatique (alignement des séquences) permet ainsi de définir des amorces dégénérées pour l'amplification de la zone hétérologue entre espèces qui correspond à la zone intergénique transcrite.

Ainsi, les amorces SEQ ID N° 1 et SEQ ID N° 31 ainsi que les autres amorces ont été définies après alignement des séquences correspondant à plus de 50 espèces d'organismes vivants (procaryotes et eucaryotes, non montré) en utilisant la redondance du code génétique. On préfère notamment les séquences SEQ ID N° 53 et SEQ ID N° 54.

Les régions amplifiées par PCR ou RT-PCR avec les amorces précitées peuvent évidemment être clonées dans différents vecteurs, afin d'être utilisées pour affiner l'analyse (en particulier pour les séquencer).

### Exemple 3 : Caractérisation des séquences des souches isolées (GroESL)

On effectue l'étude des séquences correspondant à des portions de l'opéron bactérien bicistronique GroESL. Les gènes de cet opéron sont en effet relativement homologues entre genres.

L'analyse informatique (alignement des séquences) permet ainsi de définir des amorces dégénérées pour l'amplification de la zone hétérologue entre espèces qui correspond à la zone intergénique transcrite.

Ainsi, les amorces SEQ ID N° 34 et SEQ ID N° 35 ont été définies après alignement des séquences correspondant à plus de 100 espèces d'organismes vivants (procaryotes et eucaryotes, non montré).

Les séquences SEQ ID N° 36 à SEQ ID N° 52 et en particulier SEQ ID N° 139 correspondent à des séquences complémentaires qui peuvent être utilisées pour l'amplification de microorganismes de divers genres et/ ou familles.

Les amorces SEQ ID N° 32 et SEQ ID N° 33 ont, quant à elles, été définies à partir des séquences conservées des gènes GroES et GroEL de *E. coli,* en utilisant la dégénérescence du code génétique.

### Exemple 4 : Réactions d'amplification (GroESL)

Les réactions de PCR sont effectuées selon le protocole suivant :
2 ml de bouillon de culture agité à 37°C pendant 18 h sont concentrés par centrifugation et resuspension du culot bactérien dans 30 µl d'eau distillée, puis une dilution au 1/10^{ème} de ce concentré traité à 100°C pendant 10 minutes est utilisée comme matrice pour les réactions de PCR. Les conditions de réaction sont 94 °C / 5 min, puis 25 cycles de (94 °C /30 sec, 60 °C / 45 sec, 72 °C / 30 sec), suivis d'une étape d'élongation à 72 °C pendant 7 min.

L'analyse des amplifiats permet de monter qu'on peut amplifier, en utilisant les amorces SEQ ID N° 32 et SEQ ID N° 33, la zone intergénique de différentes Entérobactéries, telles qu'*Escherichia coli*, *Enterobacter clocae*, Morganella *morganii, Serratia licquefasciens, Proteus mirabilis, Serratia marcescens, Klebsiella pneumoniae, Citrobacter freundii, Klebsiella oxytoca.* La région amplifiée varie en longueur, selon les espèces, de 400 à 500 paires de bases (pb). L'utilisation-du couple SEQ ID N° 34 et SEQ ID N° 36 donne des amplifiats de taille comprise entre 550 et 650 pb.

L'utilisation des couples (SEQ ID N° 34, SEQ ID N° 35, ou SEQ ID N° 39) / (une séquence choisie parmi les séquences SEQ ID N° 36 à SEQ ID N° 38 ou SEQ ID N° 40 à SEQ ID N° 52, ou SEQ ID N° 139) permet l'amplification de séquences spécifiques de certaines familles et espèces, et l'identification des organismes de ces familles ou espèces.

Pour les réactions d'amplification, on utilise de préférence une amorce notée « A » avec une amorce notée « B ».

Les régions amplifiées par PCR ou RT-PCR avec les amorces précitées peuvent évidemment être clonées dans différents vecteurs, afin d'être utilisées pour affiner l'analyse (en particulier pour les séquencer).

### PROTOCOLE PCR

En vue de montrer que l'utilisation de la zone intergénique des deux opérons d'intérêt comme sonde nucléique peut permettre de discriminer plusieurs espèces bactériennes, on a amplifié, par PCR directe sur suspensions bactériennes, ladite ZIG pour chaque cible. Pour les réactions d'amplification, on utilise de préférence une amorce notée « A » avec une amorce notée « B ».

2 ml de bouillon de culture agité à 37°C pendant 18 h sont concentrés par centrifugation et resuspension du culot bactérien dans 30 µl d'eau distillée, puis une dilution au 1/10^{ème} de ce concentré traité à 100°C pendant 10 minutes est utilisée comme matrice pour les réactions de PCR.

### OPERON groESL :

Les réactions de PCR, pour cette cible, sont effectuées, à une Tm variant entre 59 °C et 60°C. Les conditions de réaction sont 94 °C / 5 min, puis 25 cycles de (94°C /30 sec, 60 °C / 45 sec, 72 °C /30 sec), suivis d'une étape d'élongation à 72 °C pendant 7 min. Les régions intergéniques amplifiées sont ensuite obsevées par une électrophorèse sur gel d'agarose en utilisant une échelle 1 Kb + (Gibco BRL)

L'analyse des amplifiats permet de monter qu'on peut amplifier, en utilisant les amorces SEQ ID N° 32 et SEQ ID N° 33, la zone intergénique de différentes Entérobactéries, telles qu'*Escherichia coli, Enterobacter clocae, Morganella morganii, Serratia licquefasciens, Proteus mirabilis, Serratia marcescens, Klebsiella pneumoniae, Citrobacter freundii, Klebsiella oxytoca.* La région amplifiée varie en longueur, selon les espèces, de 400 à 500 paires de bases (pb). L'utilisation du couple SEQ ID N° 34 et SEQ ID N° 36 donne des amplifiats de taille comprise entre 550 et 650 pb.

### OPERON rpoB/C:

Les réactions de PCR , pour cette cible, sont effectuées, à une Tm variant entre 63 °C et 64°C. Les conditions de réaction sont 94 °C / 4 min, puis 30 cycles de (94 °C / 30 sec, 64 °C / 30 sec, 72 °C /3 min), suivis d'une étape d'élongation à 72 °C pendant 12 min. Les régions intergéniques amplifiées sont ensuite obsevées par une électrophorèse sur gel d'agarose en utilisant une échelle DNA molecular weight marker III (Ref : n°528552 ; Boehringer Mannheim).

L'analyse des amplifiats permet de montrer qu'on peut amplifier, en utilisant le couple d'amorce SEQ ID N° 53 et SEQ ID N° 54, la zone intergénique de différentes bactéries telles qu*'Escherichia coli, Clostridium leptum, Klebsellia oxytoca, Lactococcus lactis, Citrobacter freundii, Serratia marcescens, Proteus mirabilis, Serratia liquefaciens, Morganella morganii, Enterobacter cloacae, Ruminococcus hydrogenotrophicus.*

Des fragments d'ADN correspondants aux régions intergéniques de l'opéron rpoB/C chez différentes espèces ont été réamplifiées et analysées à partir de bandes extraites d'une préparation de gel d'agarose. Ces fragments étant préalablement purifiés avec un kit d'extraction Qiagen.

Les régions amplifiées par PCR ou RT-PCR avec les amorces précitées peuvent évidemment être clonées dans différents vecteurs, afin d'être utilisées pour affiner l'analyse (en particulier pour les séquencer).

### PROTOCOLE HYBRIDATION

En vue de tester la spécificité des produits PCR, pour les espèces sélectionnées, pour notre étude, on a réalisé des dépôts de ces ADN sur membrane de nylon en suivant un protocole de fixation de la soude (NaOH). Les concentrations en ADN, pour ces dépôts, sont indiqués sur les figures correspondantes. Ces membranes ont été hybridées en suivant le protocole du PCR DIG Probe Synthesis Kit (Roche) Cat No. 1636090. La concentration de la sonde utilisée, synthétisée suivant le même protocole, est aussi indiquée sur les figures ainsi que la température d'hybridation réalisée « over night » (18 h). La température de pr é-hybridation est de 65°C pour chaque expérience et celle-ci dure 45 min..

La détection de ce type d'hybridation avec ce type de marquage (DIG) est dite colorimétrique (marquage « froid » différent de radioactif).

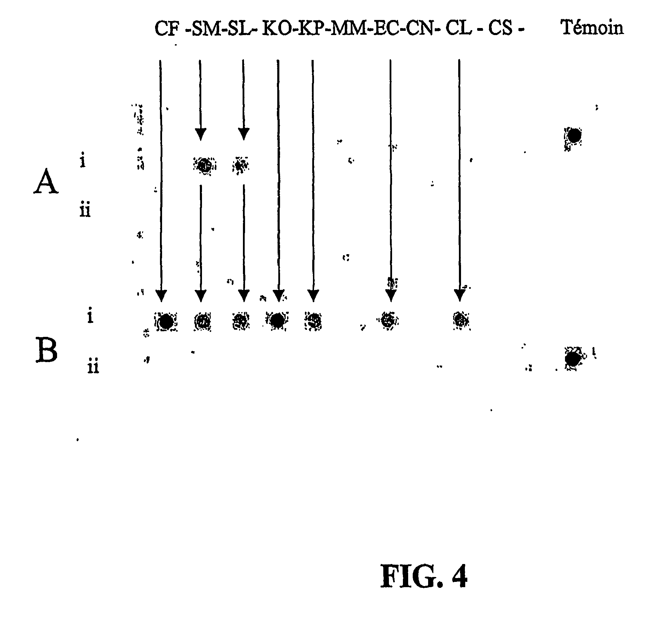

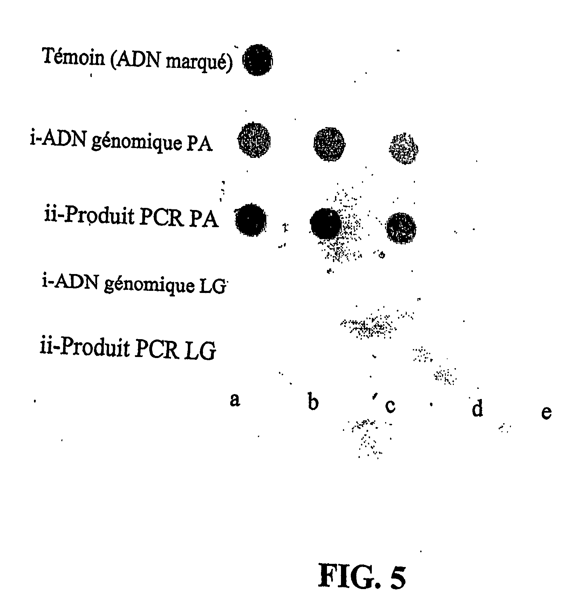

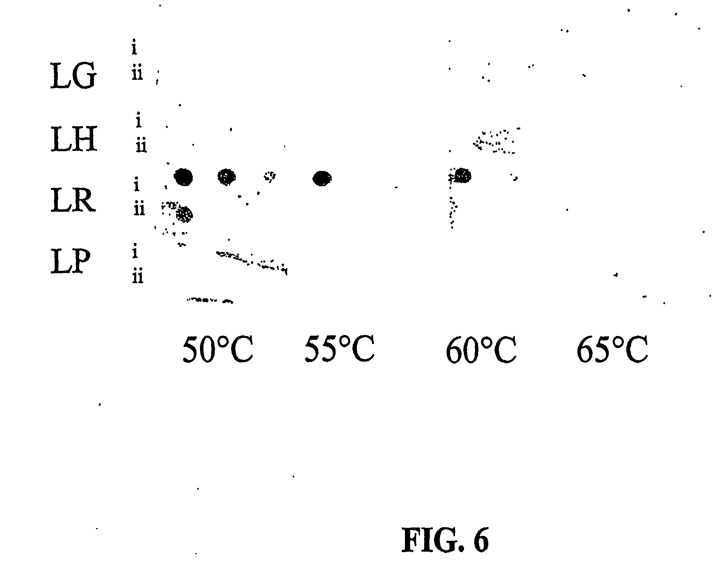

Les figures 4 à 6 montrent une spécificité de détection en fonction des organismes, bien qu'il puisse exister quelques réactions d'hybridation croisée. Ces réactions peuvent être réduites en choisissant des sondes qui soient plus courtes et situées parmi les séquences intergéniques hypervariables, telles que définies par SEQ ID N° 63 à SEQ ID N° 138 (rpoN).

Ainsi, une puce à ADN avec différentes sondes situées dans la zone intergénique permettra de reconnaître sans hésitation la présence ou l'absence d'un microorganisme, même dans le cas où il y a hybridation croisée. En effet, la présence d'un microorganisme se déduira de l'hybridation pour chacune des sondes.

Il peut donc être avantageux de définir des puces à ADN ayant des sondes spécifiques correspondant à la région intergénique de chaque microorganisme, mais également de mettre plusieurs sondes différentes pour chaque microorganisme.

### Références

{1}- Welling, et al., Applied Environmental Microbiology, **64**:3336-45, 1998: Variations of bacterial populations in human feces measured by fluorescent in situ hybridization with group-specific 16S rRNA-targeted oligonucleotide probes.
{2}- Greisen, et al., Journal of Clinical Microbiology, **32**:335-351, 1994: Staphylococcal identification using PCR amplification of 16S rDNA genes..
{3}- Jensen, et al., Applied Environmental Microbiology, **59**:945-952, 1994: Staphylococcal identification using PCR amplification of spacer regions between 16S and 23S genetic loci.
{4}- Plikaytis, et al., supra, Telenti, et al., supra: PCR strategies for Mycobacterial speciation requiring the detection of restriction site polymorphisms (RFLP) within amplified products.
{5} - Zoetendal, Akkermans and De Vos, Applied Environmental Microbiology, **64**:3854-9 (1998): Temperature gradient gel electrophoresis analysis of 16S rRNA from human fecal samples reveals stable and host-specific communities of active bacteria.
{6}- Vaitilinggom, Gendre and Brignon, Applied Environmental Microbiology **64**:1157-60, 1998: Direct detection of viable bacteria, molds, and yeast by reverse transcriptase PCR in contaminated milk samples after heat treatment.
{7}- Gendre et Brignon, Compagnie Gervais Danone, brevet # PCT/FR97/01918 WO98/18958 (1998): Method for detecting live microbiological contaminants in a food product sample.
{8}- Doré, et al., Applied Environmental Microbiology, **65**:4799-807, 1999: Direct analysis of genes encoding 16S RNA from complex commonities reveals many novel molecular species within human gut.
{9}- Goh, Chow et Hemmingsen, US Patents 5,708,160 (1998) & 5,989,521 (1999).
{10}- Emelyanov and Sinitsyn, Russian Journal of Genetics, **35**;618-627, 1999: A GnoE-based phylogenetic analysis shows very close evolutionary relationship between mitochondria and rickettsia.
{11} Brevet US 5,989,821 délivre le 23 novembre 1999
{12} "rpoB Gene Analysis as a Novel Strategy for Identificaiton of Spirochetes from the Général *Borrelia, Trepona,* and *Leptospira"* P. Renesto *et al.* CNRS UPRES-A 6020, Faculté de Médecine, 27 janvier 2000

## Revendications

1. Procédé de détection des éléments constitutifs d'une flore bactérienne dont au moins une partie des éléments possède un opéron en commun, **caractérisé en ce que** :
a) on prépare l'ADN génomique de ladite flore ou les ARNm,
b) on amplifie au moins une partie des séquences intergéniques non codantes situées dans l'opéron conservé chez au moins une partie des éléments de la flore et
c) on identifie les différentes séquences intergéniques amplifiées afin de déterminer les éléments de ladite flore,
ledit opéron étant un opéron rpoBC.

2. Procédé selon la revendication 1, **caractérisé en ce que** l'identification des séquences amplifiées est réalisée sur une puce à ADN comportant des séquences complémentaires des séquences susceptibles d'être amplifiées à partir des éléments connus de ladite flore et la mise en évidence d'éventuelles hybridations permettant d'identifier les éléments présents dans la flore.

3. Procédé selon l'une des revendications 1 ou 2, **caractérisé en ce que** les amorces destinées à amplifier la séquence intergénique sont situées dans les séquences codantes pour les gènes flanquants.

4. Procédé selon l'une des revendications 1 à 3, **caractérisé en ce que** les séquences intergéniques au moins partiellement amplifiées sont la zone intergénique transcrite entre les gènes rpoB et rpoC (ou homologues).

5. Procédé selon l'une des revendications 1 à 4, **caractérisé en ce qu'**au moins une amorce est choisie parmi les séquences SEQ ID N°1 à SEQ ID N° 31.

6. Puce à ADN **caractérisée en ce qu'**elle présente, à sa surface, des séquences complémentaires aux séquences intergéniques non codantes situées dans un opéron conservé entre différentes espèces, ledit opéron étant un opéron rpoBC.

7. Puce à ADN selon la revendication 6), **caractérisée en ce que** les séquences de plusieurs organismes, complémentaires aux séquences intergéniques non codantes situées dans ledit opéron conservé sont présentes à la surface de ladite puce.

8. Trousse de diagnostic pour la mise en oeuvre d'un procédé selon l'une des revendications 1 à 5, **caractérisée en ce qu'**elle contient des amorces dégénérées permettant l'amplification d'une ou plusieurs régions intergéniques d'un opéron conservé parmi les espèces, et de façon optionnelle, une puce à ADN selon l'une des revendications 6 ou 7.

9. Amorce pour la mise en oeuvre d'un procédé selon l'une des revendications 1 à 5, **caractérisée en ce qu'**elle est choisie parmi les séquences SEQ IDN°1 à SEQ ID N°31 et SEQ ID N° 53 à SEQ ID N°61.

10. Puce à ADN selon la revendication 6 ou 7, présentant à sa surface une pluralité d'oligonucléotides comprenant des fragments de taille supérieure à 30 bases choisis parmi les fragments des séquences SEQ ID N° 63 à SEQ ID N° 138.

## Patentansprüche

1. Verfahren zur Detektion der Bestandteile, aus welchen eine bakterielle Flora besteht, bei welchen wenigstens ein Teil der Bestandteile ein gemeinsames Operon aufweist, **dadurch gekennzeichnet, dass**:
a) man die genomische DNA der Flora oder die mRNAs präpariert,
b) man wenigstens einen Teil der intergenischen, nicht-kodierenden Sequenzen, die in dem bei wenigstens einem Teil der Bestandteile der Flora konservierten Operon gelegen sind, amplifiziert und
c) man die unterschiedlichen amplifizierten intergenischen Sequenzen identifiziert, um die Bestandteile der Flora zu bestimmen,
wobei das Operon ein rpoBC-Operon ist.

2. Verfahren nach Anspruch 1, **dadurch gekennzeichnet, dass** die Identifizierung der amplifizierten Sequenzen auf einem DNA-Chip erfolgt, welcher Sequenzen umfasst, die zu Sequenzen komplementär sind, die ausgehend von bekannten Bestandteilen der Flora amplifiziert werden können, und wobei der Nachweis von gegebenenfalls erfolgenden Hybridisierungen erlaubt, die in der Flora vorhandenen Bestandteile zu identifizieren.

3. Verfahren nach einem der Ansprüche 1 oder 2, **dadurch gekennzeichnet, dass** die Primer, welche dazu bestimmt sind, die intergenische Sequenz zu amplifizieren, in den die flankierenden Gene kodierenden Sequenzen gelegen sind.

4. Verfahren nach einem der Ansprüche 1 bis 3, **dadurch gekennzeichnet, dass** die wenigstens teilweise amplifizierten intergenischen Sequenzen die transkribierte intergenische Zone zwischen den Genen rpoB und rpoC (oder homologen Genen) sind.

5. Verfahren nach einem der Ansprüche 1 bis 4, **dadurch gekennzeichnet, dass** wenigstens ein Primer unter den Sequenzen SEQ ID Nr. 1 bis SEQ ID Nr. 31 ausgewählt wird.

6. DNA-Chip, **dadurch gekennzeichnet, dass** er auf seiner Oberfläche Sequenzen aufweist, welche komplementär sind zu den intergenischen, nicht-kodierenden Sequenzen, die in einem zwischen unterschiedlichen Spezies konservierten Operon gelegen sind, wobei das Operon ein rpoBC-Operon ist.

7. DNA-Chip nach Anspruch 6, **dadurch gekennzeichnet, dass** die Sequenzen von mehreren Organismen, die komplementär sind zu den intergenischen, nicht-kodierenden Sequenzen, die in dem konservierten Operon gelegen sind, auf der Oberfläche des Chips vorhanden sind.

8. Diagnostischer Kit für das Ausführen eines Verfahrens nach einem der Ansprüche 1 bis 5, **dadurch gekennzeichnet, dass** er degenerierte Primer, welche die Amplifizierung von einer oder mehreren intergenischen Regionen eines zwischen den Spezies konservierten Operons erlauben, und gegebenenfalls einen DNA-Chip nach einem der Ansprüche 6 oder 7 umfasst.

9. Primer für das Ausführen eines Verfahrens nach einem der Ansprüche 1 bis 5, **dadurch gekennzeichnet, dass** er ausgewählt wird unter den Sequenzen SEQ ID Nr. 1 bis SEQ ID Nr. 31 und SEQ ID Nr. 53 bis SEQ ID Nr. 61.

10. DNA-Chip nach Anspruch 6 oder 7, welcher auf seiner Oberfläche eine Mehrzahl von Oligonukleotiden, umfassend Fragmente mit einer Größe über 30 Basen, welche unter den Fragmenten mit den Sequenzen SEQ ID Nr. 63 bis SEQ ID Nr. 138 ausgewählt werden, aufweist.

## Claims

1. Method for detecting the elements constituting a bacterial flora, at least some of the elements of which have an operon in common, **characterized in that**:
a) the genomic DNA of said flora or the mRNAs is (are) prepared,
b) at least some of the noncoding intergenic sequences located in the operon conserved in at least some of the elements of the flora are amplified, and
c) the various intergenic sequences amplified are identified in order to determine the elements of said flora,
said operon being an rpoBC operon.

2. Method according to Claim 1, **characterized in that** the identification of the amplified sequences is carried out on a DNA kit comprising sequences complementary to the sequences liable to be amplified from the known elements of said flora, and the demonstration of possible hybridizations making it possible to identify the elements present in the flora.

3. Method according to either of Claims 1 and 2, **characterized in that** the primers intended to amplify the intergenic sequence are located in the coding sequences of the flanking genes:

4. Method according to one of Claims 1 to 3, **characterized in that** the intergenic sequences at least partially amplified are the intergenic region transcribed between the rpoB and rpoC genes (or homologous genes).

5. Method according to one of Claims 1 to 4, **characterized in that** at least one primer is chosen from the sequences SEQ ID No. 1 to SEQ ID No. 31.

6. DNA chip, **characterized in that** it has, at its surface, sequences complementary to the noncoding intergenic sequences located in an operon which is conserved between various species, said operon being an rpoBC operon.

7. DNA chip according to Claim 6, **characterized in that** the sequences of several organisms, complementary to the noncoding intergenic sequences located in said conserved operon, are present at the surface of said chip.

8. Diagnostic kit for carrying out a method according to one of Claims 1 to 5, **characterized in that** it contains degenerate primers for amplifying one or more intergenic regions of an operon which is conserved among species and, optionally, a DNA chip according to either of Claims 6 and 7.

9. Primer for carrying out a method according to one of Claims 1 to 5, **characterized in that** it is chosen from the sequences SEQ ID No. 1 to SEQ ID No. 31 and SEQ ID No. 53 to SEQ ID No. 61.

10. DNA chip according to Claim 6 or 7, having, at its surface, a plurality of oligonucleotides comprising fragments more than 30 bases long, chosen from the fragments of the sequences SEQ ID No. 63 to SEQ ID No. 138.


### LISTE DE SEQUENCES

<110> UNIVERSITE D'AUVERGNE / DIGESTAR
<120> PROCEDE DE DETECTION DE MICRO-ORGANISMES
<130> D18287
<150> FR 00/09600
   <151> 2000-07-21
<150> FR 00/12524
   <151> 2000-10-02
<160> 189
<170> PatentIn Ver. 2.1
<210> 1
   <211> 26
   <212> ADN
   <213> Séquence artificielle
<220>
   <223> Description de la séquence artificielle: AMORCE PERMETTANT L'AMPLIFICATION DE LA REGION INTERGENIQUE DE L'OPERON rpoBC DE MICRO-ORGANISMES
<400> 1
   ggngayaary tngcnggnag ncaygg 26
<210> 2
   <211> 26
   <212> ADN
   <213> Séquence artificielle
<220>
   <223> Description de la séquence artificielle: AMORCE PERMETTANT L'AMPLIFICATION DE LA REGION INTERGENIQUE DE L'OPERON rpoBC DE MICRO-ORGANISMES
<400> 2
   ggngayaary tngcnggncg ncaygg 26
<210> 3
   <211> 26
   <212> ADN
   <213> Séquence artificielle
<220>
   <223> Description de la séquence artificielle: AMORCE PERMETTANT L'AMPLIFICATION DE LA REGION INTERGENIQUE DE L'OPERON rpoBC DE MICRO-ORGANISMES
<400> 3
   ggngayaary tngcnaayag ncaygg 26
<210> 4
   <211> 26
   <212> ADN
   <213> Séquence artificielle
<220>
   <223> Description de la séquence artificielle: AMORCE PERMETTANT L'AMPLIFICATION DE LA REGION INTERGENIQUE DE L'OPERON rpoBC DE MICRO-ORGANISMES
<400> 4
   ggngayaary tngcnaaycg ncaygg 26
<210> 5
   <211> 26
   <212> ADN
   <213> Séquence artificielle
<220>
   <223> Description de la séquence artificielle: AMORCE PERMETTANT L'AMPLIFICATION DE LA REGION INTERGENIQUE DE L'OPERON rpoBC DE MICRO-ORGANISMES
<400> 5
   ggngayaara tggcnggnmg ncaygg 26
<210> 6
   <211> 26
   <212> ADN
   <213> Séquence artificielle
<220>
   <223> Description de la séquence artificielle: AMORCE PERMETTANT L'AMPLIFICATION DE LA REGION INTERGENIQUE DE L'OPERON rpoBC DE MICRO-ORGANISMES
<400> 6
   ggngayaart tygcntcnmg ncaygg 26
<210> 7
   <211> 26
   <212> ADN
   <213> Séquence artificielle
<220>
   <223> Description de la séquence artificielle: AMORCE PERMETTANT L'AMPLIFICATION DE LA REGION INTERGENIQUE DE L'OPERON rpoBC DE MICRO-ORGANISMES
<400> 7
   ggngayaart tygcnagymg ncaygg 26
<210> 8
   <211> 26
   <212> ADN
   <213> Séquence artificielle
<220>
   <223> Description de la séquence artificielle: AMORCE PERMETTANT L'AMPLIFICATION DE LA REGION INTERGENIQUE DE L'OPERON rpoBC DE MICRO-ORGANISMES
<400> 8
   ggngayaart tygcnacnmg ncaygg 26
<210> 9
   <211> 26
   <212> ADN
   <213> Séquence artificielle
<220>
   <223> Description de la séquence artificielle: AMORCE PERMETTANT L'AMPLIFICATION DE LA REGION INTERGENIQUE DE L'OPERON rpoBC DE MICRO-ORGANISMES
<400> 9
   aaygcngayt tygayggnga ycarat 26
<210> 10
   <211> 26
   <212> ADN
   <213> Séquence artificielle
<220>
   <223> Description de la séquence artificielle: AMORCE PERMETTANT L'AMPLIFICATION DE LA REGION INTERGENIQUE DE L'OPERON rpoBC DE MICRO-ORGANISMES
<400> 10
   aaygcngayt tygayggnca ratggc 26
<210> 11
   <211> 26
   <212> ADN
   <213> Séquence artificielle
<220>
   <223> Description de la séquence artificielle: AMORCE PERMETTANT L'AMPLIFICATION DE LA REGION INTERGENIQUE DE L'OPERON rpoBC DE MICRO-ORGANISMES
<400> 11
   aaygcngayt tygayggnga ygarat 26
<210> 12
   <211> 26
   <212> ADN
   <213> Séquence artificielle
<220>
   <223> Description de la séquence artificielle: AMORCE PERMETTANT L'AMPLIFICATION DE LA REGION INTERGENIQUE DE L'OPERON rpoBC DE MICRO-ORGANISMES
<400> 12
   ggnggncarm gnttyggnga ratgga 26
<210> 13
   <211> 26
   <212> ADN
   <213> Séquence artificielle
<220>
   <223> Description de la séquence artificielle: AMORCE PERMETTANT L'AMPLIFICATION DE LA REGION INTERGENIQUE DE L'OPERON rpoBC DE MICRO-ORGANISMES
<400> 13
   ggnggncayg gnttyggnga ratgga 26
<210> 14
   <211> 26
   <212> ADN
   <213> Séquence artificielle
<220>
   <223> Description de la séquence artificielle: AMORCE PERMETTANT L'AMPLIFICATION DE LA REGION INTERGENIQUE DE L'OPERON rpoBC DE MICRO-ORGANISMES
<400> 14
   ggnggncarw snttyggnga ratgga 26
<210> 15
   <211> 26
   <212> ADN
   <213> Séquence artificielle
<220>
   <223> Description de la séquence artificielle: AMORCE PERMETTANT L'AMPLIFICATION DE LA REGION INTERGENIQUE DE L'OPERON rpoBC DE MICRO-ORGANISMES
<400> 15
   ggnggnntnm gnttyggnga ratgga 26
<210> 16
   <211> 26
   <212> ADN
   <213> Séquence artificielle
<220>
   <223> Description de la séquence artificielle: AMORCE PERMETTANT L'AMPLIFICATION DE LA REGION INTERGENIQUE DE L'OPERON rpoBC DE MICRO-ORGANISMES
<400> 16
   ggnaarcgng tngaytaytc nggnmg 26
<210> 17
   <211> 26
   <212> ADN
   <213> Séquence artificielle
<220>
   <223> Description de la séquence artificielle: AMORCE PERMETTANT L'AMPLIFICATION DE LA REGION INTERGENIQUE DE L'OPERON rpoBC DE MICRO-ORGANISMES
<400> 17
   ggnaarcgng tngaytayag nggnmg 26
<210> 18
   <211> 26
   <212> ADN
   <213> Séquence artificielle
<220>
   <223> Description de la séquence artificielle: AMORCE PERMETTANT L'AMPLIFICATION DE LA REGION INTERGENIQUE DE L'OPERON rpoBC DE MICRO-ORGANISMES
<400> 18
   ggnaaragng tngaytaytc nggnmg 26
<210> 19
   <211> 26
   <212> ADN
   <213> Séquence artificielle
<220>
   <223> Description de la séquence artificielle: AMORCE PERMETTANT L'AMPLIFICATION DE LA REGION INTERGENIQUE DE L'OPERON rpoBC DE MICRO-ORGANISMES
<400> 19
   ggnaaragng tngaytayag nggnmg 26
<210> 20
   <211> 26
   <212> ADN
   <213> Séquence artificielle
<220>
   <223> Description de la séquence artificielle: AMORCE PERMETTANT L'AMPLIFICATION DE LA REGION INTERGENIQUE DE L'OPERON rpoBC DE MICRO-ORGANISMES
<400> 20
   ggnaarcgng gngaytaytc ngtnmg 26
<210> 21
   <211> 26
   <212> ADN
   <213> Séquence artificielle
<220>
   <223> Description de la séquence artificielle: AMORCE PERMETTANT L'AMPLIFICATION DE LA REGION INTERGENIQUE DE L'OPERON rpoBC DE MICRO-ORGANISMES
<400> 21
   ggnaarcgng gngaytayag ngtnmg 26
<210> 22
   <211> 26
   <212> ADN
   <213> Séquence artificielle
<220>
   <223> Description de la séquence artificielle: AMORCE PERMETTANT L'AMPLIFICATION DE LA REGION INTERGENIQUE DE L'OPERON rpoBC DE MICRO-ORGANISMES
<400> 22
   ggnaaragng gngaytaytc ngtnmg 26
<210> 23
   <211> 26
   <212> ADN
   <213> Séquence artificielle
<220>
   <223> Description de la séquence artificielle: AMORCE PERMETTANT L'AMPLIFICATION DE LA REGION INTERGENIQUE DE L'OPERON rpoBC DE MICRO-ORGANISMES
<400> 23
   ggnaaragng gngaytayag ngtnmg 26
<210> 24
   <211> 26
   <212> ADN
   <213> Séquence artificielle
<220>
   <223> Description de la séquence artificielle: AMORCE PERMETTANT L'AMPLIFICATION DE LA REGION INTERGENIQUE DE L'OPERON rpoBC DE MICRO-ORGANISMES
<400> 24
   ggnaarcgng tngayttytc nggnmg 26
<210> 25
   <211> 26
   <212> ADN
   <213> Séquence artificielle
<220>
   <223> Description de la séquence artificielle: AMORCE PERMETTANT L'AMPLIFICATION DE LA REGION INTERGENIQUE DE L'OPERON rpoBC DE MICRO-ORGANISMES
<400> 25
   ggnaarcgng tngayttyag nggnmg 26
<210> 26
   <211> 26
   <212> ADN
   <213> Séquence artificielle
<220>
   <223> Description de la séquence artificielle: AMORCE PERMETTANT L'AMPLIFICATION DE LA REGION INTERGENIQUE DE L'OPERON rpoBC DE MICRO-ORGANISMES
<400> 26
   ggnaaragng tngayttytc nggnmg 26
<210> 27
   <211> 26
   <212> ADN
   <213> Séquence artificielle
<220>
   <223> Description de la séquence artificielle: AMORCE PERMETTANT L'AMPLIFICATION DE LA REGION INTERGENIQUE DE L'OPERON rpoBC DE MICRO-ORGANISMES
<400> 27
   ggnaaragng tngayttyag nggnmg 26
<210> 28
   <211> 26
   <212> ADN
   <213> Séquence artificielle
<220>
   <223> Description de la séquence artificielle: AMORCE PERMETTANT L'AMPLIFICATION DE LA REGION INTERGENIQUE DE L'OPERON rpoBC DE MICRO-ORGANISMES
<400> 28
   ggnaarcgng tngayttytc ngcnmg 26
<210> 29
   <211> 26
   <212> ADN
   <213> Séquence artificielle
<220>
   <223> Description de la séquence artificielle: AMORCE PERMETTANT L'AMPLIFICATION DE LA REGION INTERGENIQUE DE L'OPERON rpoBC DE MICRO-ORGANISMES
<400> 29
   ggnaarcgng tngayttyag ngcnmg 26
<210> 30
   <211> 26
   <212> ADN
   <213> Séquence artificielle
<220>
   <223> Description de la séquence artificielle: AMORCE PERMETTANT L'AMPLIFICATION DE LA REGION INTERGENIQUE DE L'OPERON rpoBC DE MICRO-ORGANISMES
<400> 30
   ggnaaragng tngayttytc ngcnmg 26
<210> 31
   <211> 26
   <212> ADN
   <213> Séquence artificielle
<220>
   <223> Description de la séquence artificielle: AMORCE PERMETTANT L'AMPLIFICATION DE LA REGION INTERGENIQUE DE L'OPERON rpoBC DE MICRO-ORGANISMES
<400> 31
   ggnaaragng tngayttyag ngcnmg 26
<210> 32
   <211> 26
   <212> ADN
   <213> Séquence artificielle
<220>
   <223> Description de la séquence artificielle: Amorce correspondant à un motif protéique de HSP10 d'Escherichia Coli.
<400> 32
   ctggaygtka arrtnggyga yatygt 26
<210> 33
   <211> 26
   <212> ADN
   <213> Séquence artificielle
<220>
   <223> Description de la séquence artificielle: Amorce correspondant à un motif protéique de HSP10 d'Escherichia Coli.
<400> 33
   annacngtng crgtrgtggt rccgtc 26
<210> 34
   <211> 26
   <212> ADN
   <213> Séquence artificielle
<220>
   <223> Description de la séquence artificielle: Amorce consensus (UNI-ADEG 1)
<400> 34
   ggngayggna cnacnacngc nacnnt                                    26
<210> 35
   <211> 26
   <212> ADN
   <213> Séquence artificielle
<220>
   <223> Description de la séquence artificielle: Amorce consensus (UNI-ADEG 2)
<400> 35
   ggngayggna cnacnacntg ntcnnt                                    26
<210> 36
   <211> 26
   <212> ADN
   <213> Séquence artificielle
<220>
   <223> Description de la séquence artificielle: Séquence consensus pour la détection des entérobactéries (ENT-BNEW).
<400> 36
   aanmttcgtc cnytrcanga ycgngt                                    26
<210> 37
   <211> 26
   <212> ADN
   <213> Séquence artificielle
<220>
   <223> Description de la séquence artificielle: Séquence consensus pour la détection des clostridia (CLO-BNEW2)
<400> 37
   atnarrccay twggwgaymg ngtwgt                                    26
<210> 38
   <211> 26
   <212> ADN
   <213> Séquence artificielle
<220>
   <223> Description de la séquence artificielle: Séquence consensus pour la détection des bifidobactéries (BIF-BNEW).
<400> 38
   aarccrctcg aggacmrnrt nstsgt 26
<210> 39
   <211> 26
   <212> ADN
   <213> Séquence artificielle
<220>
   <223> Description de la séquence artificielle: Séquence consensus pour la détection des Lactococcus (UNI-A3).
<400> 39
   ggngayggna cnaanacngc nacnnt 26
<210> 40
   <211> 26
   <212> ADN
   <213> Séquence artificielle
<220>
   <223> Description de la séquence artificielle: Séquence consensus pour la détection des Bifidobacterium et Mycobacterium (BIF-BNEW2).
<400> 40
   atcaagccnc tmgrrgacmr srtnst 26
<210> 41
   <211> 26
   <212> ADN
   <213> Séquence artificielle
<220> <223> Description de la séquence artificielle: Séquence consensus pour la détection des Helicobacter (HEL-BNEW).
<400> 41
   ntncanccnt tnggnganag ngtntt 26
<210> 42
   <211> 26
   <212> ADN
   <213> Séquence artificielle
<220> <223> Description de la séquence artificielle: Séquence consensus pour la détection des Campylobacter (CAM-BNEW).
<400> 42
   ntncanccnt tnggnaancg ngtnct 26
<210> 43
   <211> 26
   <212> ADN
   <213> Séquence artificielle
<220>
   <223> Description de la séquence artificielle: Séquence consensus pour la détection des bacteroïdes (BACT-BNEW).
<400> 43
   ntnaanccnt tngcngancg ngtnct 26
<210> 44
   <211> 26
   <212> ADN
   <213> Séquence artificielle
<220>
   <223> Description de la séquence artificielle: Séquence consensus pour la détection des Chlamydia (CHLA-BNEW).
<400> 44
   ntnaanccnt tnggnganag natntt 26
<210> 45
   <211> 26
   <212> ADN
   <213> Séquence artificielle
<220>
   <223> Description de la séquence artificielle: Séquence consensus pour la détection des Mycoplasma (MYCP-BNEW).
<400> 45
   ntnaaaccnn tnggnaancg ngtnat 26
<210> 46
   <211> 26
   <212> ADN
   <213> Séquence artificielle
<220>
   <223> Description de la séquence artificielle: Séquence consensus pour la détection des Staphylococcus (STA-BNEW).
<400> 46
   ntnaaaccnn tnggnaancg ngtnat 26
<210> 47
   <211> 26
   <212> ADN
   <213> Séquence artificielle
<220>
   <223> Description de la séquence artificielle: Séquence consensus pour la détection des Lactoccocus et Streptococcus (LACC-BNEW).
<400> 47
   ttgaaaccnt tagngraycg ygtrst 26
<210> 48
   <211> 26
   <212> ADN
   <213> Séquence artificielle
<220>
   <223> Description de la séquence artificielle: Séquence consensus pour la détection des Lactobacillus et Bacillus (LACB-BNEW).
<400> 48
   ttamarccaw tmggngatcg ngtnrt 26
<210> 49
   <211> 26
   <212> ADN
   <213> Séquence artificielle
<220>
   <223> Description de la séquence artificielle: Séquence consensus pour la détection des Clostridium (CLO-BNEW3).
<400> 49
   atnanaccan tnggngacag ngtngt 26
<210> 50
   <211> 26
   <212> ADN
   <213> Séquence artificielle
<220>
   <223> Description de la séquence artificielle: Séquence consensus pour la détection des Enterobacteriaceae, Pasteurella, Haemophilus (ENT-BNEW2).
<400> 50
   ntncgnccnt tncangancg ngtnat 26
<210> 51
   <211> 26
   <212> ADN
   <213> Séquence artificielle
<220>
   <223> Description de la séquence artificielle: Séquence consensus pour la détection des Neisseria, Legionella (LEG-BNEW).
<400> 51
   ntncgnccnt tncangancg ngtngt 26
<210> 52
   <211> 26
   <212> ADN
   <213> Séquence artificielle
<220>
   <223> Description de la séquence artificielle: Séquence consensus pour la détection des Aeromonas et Bordetella (AER-BNEW).
<400> 52
   ntncgnccnc tncangancg ngtnat 26
<210> 53
<211> 26
<212> ADN
<213> Sequence artificielle
<220>
   <223> Amorce
<220>
   <223> n signifie a,g,c ou t/u
<400> 53
   ggnggncann snttyggnga ratgga 26
<210> 54
<211> 26
<212> ADN
<213> Sequence artificielle
<220>
   <223> Amorce
<220>
   <223> n signifie a,g,c ou t/u
<400> 54
   aaygcngayt tygayggnga ysarat 26
<210> 55
<211> 26
<212> ADN
<213> Sequence artificielle
<220>
   <223> Amorce
<220>
   <223> n signifie a,g,c ou t/u
<400> 55
   ggnggncarm gnttyggnga ratgga 26
<210> 56
<211> 26
<212> ADN
<213> Séquence artificielle
<220>
   <223> Amorce
<220>
   <223> n signifie a,g,c ou t/u
<400> 56
   ggnggncayg gnttyggnga ratgga 26
<210> 57
<211> 26
<212> ADN
<213> Sequence artificielle
<220>
   <223> Amorce
<220>
   <223> n signifie a,g,c ou t/u
<400> 57
   ggnggncarw snttyggnga ratgga 26
<210> 58
<211> 26
<212> ADN
<213> Sequence artificielle
<220>
   <223> Amorce
<220>
   <223> n signifie a,g,c ou t/u
<400> 58
   ggnggnntnm gnttyggnga ratgga 26
<210> 59
<211> 26
<212> ADN
<213> Sequence artificielle
<220>
   <223> Amorce
<220>
   <223> n signifie a,g,c ou t/u
<400> 59
   aaygcngayt tygayggnga ycarat 26
<210> 60
<211> 26
<212> ADN
<213> Sequence artificielle
<220>
   <223> Amorce
<220>
   <223> n signifie a,g,c ou t/u
<400> 60
   aaygcngayt tygayggnca ratggc 26
<210> 61
<211> 26
<212> ADN
<213> Séquence artificielle
<220>
   <223> Amorce
<220>
   <223> n signifie a,g,c ou t/u
<400> 61
   aaygcngayt tygayggnga ygarat 26
<210> 62
<211> 26
<212> ADN
<213> Sequence artificielle
<220>
   <223> Amorce
<220>
   <223> n signifie a,g,c ou t/u
<400> 62
   atytsrtcnc crtcraartc ngcrtt 26
<210> 63
<211> 333
<212> ADN
<213> Lactobacillus reuteri
<400> 63
   cttgttaagg aattacaagc attaggtctt gatatgaagg ttcttgatgg taacaacaag 60
   gaaattcagt taaagaacat ggacgaagat gatgatgaag ttgtaaatgt tgatgcatta 120
   gctaaatatg cagaagaaca taaaacagac gataagaaga acgaagaaga aaacaagtct 180
   gaagcaactt caacaactac cgatgacaaa actaatcaaa attaatattt aggttgctac 240
   ggtttactga aagaaggagg aacatccttt gattgatgtc aataaatttg aaagtatgca 300
   gatcggtctg gcatctccag ataagatccg tag 333
<210> 64
<211> 338
<212> ADN
<213> Bacillus subtilis
<400> 64
   cttgttaagg aacttcaatc attaggtttg gatattcgtg ttcttgatat gaatcataat 60
   gaaattgaac ttcgtgatat ggatgaagat tcaagtgaac acttaaacat tgattcattg 120
   tcacgtatgg cagaagaaca agaaaagaag aagttagccg aagaaactgg aaaatcagaa 180
   gataagaaag aaaacaagaa agatgcagat aagctagtag ctcctgcaga tgaatctgac 240
   gacgaagttt ctaaatagta ggaggttaaa cttttgatcg acgtaaataa atttgaaagt 300
   atgcaaattg gtcttgcatc acctaacaag atcagaag 338
<210> 65
<211> 329
<212> ADN
<213> Lactobacillus gaseri
<400> 65
   cttgttaagg aacttcaatc cttaggtttg gatattaaag tcttagatat ggaccacaag 60
   gaaattgaat tacgtgacat ggatgatgat tctaatgatc acttcaacat tgacacttta 120
   tctaagcttg ctgaacaaca agaaaagaag aagttagccg aagaagctgc aaagaaagat 180
   gataagtcag ccgaacctgt agatcagagt gattcttcaa cttcatctga tgataaggtt 240
   tctaagtaat aggaggttaa acttttgatc gacgtaaata agtttgaaag tatgcaaatt 300
   ggtttggctt ctccaaacaa gatcagaag 329
<210> 66
<211> 296
<212> ADN
<213> Lactobacillus paracasei
<400> 66
   cttgtcaaag aattgcaagc actgggtctg gatatgaagg tccttggcgc ggataaaaaa 60
   gaaattgaac tgcgggacat ggacgacgac gaggatgata ttgtttctgt cgatgccttg 120
   gcgaagtttg ctgctcagca ggaagaaaag aaggctcacg aagccgcagc acaagcaact 180
   gacggtaagt ctgccaacag taccgacgat aagaaatagg aggttagccc tttgattgat 240
   gtcaataagt ttgaaagtat gcaaatcggc ttagcctcgc cagataaaat ccgtag 296
<210> 67
<211> 386
<212> ADN
<213> LACTOCOCCUS LACTIS
<400> 67
   ttggttaaag agttacaatc acttggtctt gatatgaaag tccttgatgc tgaccgtaat 60
   gttcttgact tacgtgaatt ggatgaagat gaagtaatga ctcgtccaga taatacagaa 120
   attactcctg aaatgcttga agcacaggaa gctattgttg cacaagcaga agctgaagaa 180
   gaagctttga ttaacgctga tactgaaaaa taagattttg taattaatat tttgagatag 240
   atttactgac aaaaatttct gtcagtaaat ctctaatctc ataatcgtct agcgttaaat 300
   ttattagaag tggagaaaga attggttgat gtaaataaat ttgagagtat gcgtattggt 360
   atcgcatctc cacaaaaaat tcgtta 386
<210> 68
<211> 344
<212> ADN
<213> STREPTOCOCCUS PYOGENES
<400> 68
   cttgtaaaag aattgcaatc gcttggtctt gatatgcgtg tgcttgacga ggatgataat 60
   gaagtggaac ttcgtgatct tgatgaaggt gaagacgatg acattatgca tgttgacgat 120
   ctcgagaagg cacgtgaaaa acaagctcaa gaaactcaag aagtttctga aacaactgac 180
   gaaaaataag caatcaattc ttattaaata attatttact ggtctggggc aaaggcccca 240
   ggaactggta aagtcatcaa aggcagaaag gtaaaactag tggttgacgt aaatcgtttt 300
   aaaagtatgc aaatcacatt agcctcacca agtaaggtcc gttc 344
<210> 69
<211> 318
<212> ADN
<213> Lactobacillus helveticus
<400> 69
   ttaatcaaag aacttcaaag cttaggtatg gatgtcaaaa tcctttctgg tgatgaagaa 60
   gaaatagaaa tgagagattt agaagacgaa gaagatgcga aacaagctga cggcctggca 120
   ttatcaggtg atgaagagcc ggaagaaaca gcatctgcag acgttgaacg cgatgtagta 180
   acaaaagaat aatctctagt tataaaggca agtgacatcg gttaatccga agataaaaag 240
   ggaggtaggc cccttgctag atgtgaacaa ttttgagtat atgaacatcg gtcttgcttc 300
   accagataaa atccgttc 318
<210> 70
<211> 318
<212> ADN
<213> BACILLUS SUBTILIS
<400> 70
   ttaatcaaag aacttcaaag cttaggtatg gatgtcaaaa tcctttctgg tgatgaagaa 60
   gaaatagaaa tgagagattt agaagacgaa gaagatgcga aacaagctga cggcctggca 120
   ttatcaggtg atgaagagcc ggaagaaaca gcatctgcag acgttgaacg cgatgtagta 180
   acaaaagaat aatctctagt tataaaggca agtgacatcg gttaatccga agataaaaag 240
   ggaggtaggc cccttgctag atgtgaacaa ttttgagtat atgaacatcg gtcttgcttc 300
   accagataaa atccgttc 318
<210> 71
<211> 304
<212> ADN
<213> BACILLUS HALODURANS
<400> 71
   cttataaaag agctacagtc tctcggtatg gacgtcaaga tgctatcaag tactgaggaa 60
   gagattgaaa tgaaagagct tgatgatgag gatgaacaag caagcgacaa attgaacttg 120
   aatattgatt caacagaatc aaatgtttaa tcagctgaaa ggggagcagt cccctttcac 180
   ttgctcttta aattcgttac ctgcttttgg acatggaaat cataagggag gttggcccct 240
   tgatagacgt aaacaatttt gagtacatga aaattggtct tgcttcacca aataaaattc 300
   gttc 304
<210> 72
<211> 363
<212> ADN
<213> STAPHYLOCOCCUS AUREUS
<400> 72
   ttgatgaaag aattacaaag tttaggttta gatgtaaaag ttatggatga gcaagataat 60
   gaaatcgaaa tgacagacgt tgatgacgat gatgttgtag aacgcaaagt agatttacaa 120
   caaaatgatg ctcctgaaac acaaaaagaa gttactgatt aatacgcaat ttacaaaaca 180
   ggcaaaaaga tactaagctg aattttattg atgattcagt ttagtacttt aagccatttt 240
   aaataaatgc aaatcaatca aatagcacag ctaatctaaa ttgaaggagg taggctcctt 300
   gattgatgta aataatttcc attatatgaa aataggattg gcttcacctg aaaaaatccg 360
   ttc 363
<210> 73
<211> 352
<212> ADN
<213> Clostridium spiroforme
<400> 73
   ttaaagaaag agttacaagc acttgcattg gatgtacgtt tgttagatga aaatgataat 60
   gaagttgata tgcgtaatat tgaagaagag gaacatcgtt tcccgcgtag cattgataaa 120
   gatgaagtaa ttgaaactcc aaaaactgat gatgaagttt ccgaagaaat tactgaagat 180
   gatttaaatg tagaagaatg tgacgtatgt gaagaagata actttgagga caatgacttc 240
   gaagacaatg atattgaaga aagtgaatca ttataggagg aattacgatg gcaaatacaa 300
   ataaattctc agcgattcaa attggtttag cttcgcttca gaagattcgc ga 352
<210> 74
<211> 358
<212> ADN
<213> Clostridium leptum
<400> 74
   ctcattaagg agcttcagtc cctgggcctg gatgtaaagg tgctggataa ggatgagcag 60
   gagatcgacc taaagcagaa ctttgacgac gatgacgata tcggcttgaa cgacggcggc 120
   accattctgg aggaggatga agtcatgacc tccatggatg gctacaccct ggaggacgat 180
   ccggacgata acaacatgtt tgacgattcc ggcttttttg acgaagacgg agacgatctt 240
   ttggattttg attccattgc aagtgatatt cgtgaagaat aaggaggggc gataggatgg 300
   agtttaacgt ttttgagtca attaaaatcg gactggcctc tccggataaa attcgaga 358
<210> 75
<211> 376
<212> ADN
<213> Clostridium nexile
<400> 75
   ctcctgaaag aacttcagtc actgggactt gacgtgagag tattgcgtga agatcagaca 60
   gaagttgaga ttatggagac aatcgattac ggtgaaacag atttacattc aattattgaa 120
   ggagacagaa gatacaattc tgagaatgaa tcttatggag aacatggttt cagtcagcag 180
   gaatttgcag gcgaggaact tgtggatgta gaggaagatg aatttgatga accggatgat 240
   atcgattttg acgatatgtt agacgaagaa taggaggatt gccaataatg ccagtaacaa 300
   ataatgaacc agcataccag ccgatgactt ttgatgcgat caaaatcggt ttggcgtcac 360
   ctgaaaaaat cttgga 376
<210> 76
<211> 391
<212> ADN
<213> Ruminococcus hydrogenotrophicus
<400> 76
   ctcttaaaag aacttcagtc cctgggtctg gacgtgagag tcctcaacga agaccagacc 60
   gaggtggaga tcatggagag cgtggattac ggtgatacag atctgcactc catcattgag 120
   ggagatcgtc atcgttcgca ggatgagtcc tacggagcaa tgggatatac gaagcaggaa 180
   ttttccggtg aagagctggt agacatcgac gagagtgaag acgacagcga agacgaagat 240
   gaagatttga ttgaattgga agattctctt gacagagaag agtagaaagg ggtaagaaac 300
   aaatggcaga aatgaacaac aatgaaacct atcagccaat gactttcgat gccatcaaaa 360
   tcggactggc gtcccctgag aaaatcagag a 391
<210> 77
<211> 182
<212> ADN
<213> CHLAMYDIA MURIDARUM
<400> 77
   ttgattaaag aaatgcaagg tctagggctc gatgttcgcc ctatggtagt agatgcttaa 60
   aaaacacttg ttggagataa gttaatgttc aaagaaggtt ctcgagacga tgcagcccta 120
   gcaaaagaag ggttgtttga taagttagaa attgggattg cttcagatgt gactattcgc 180
   ga 182
<210> 78
<211> 182
<212> ADN
<213> CHLAMYDIA TRACHOMATIS
<400> 78
   ttgattaaag aaatgcaagg tctagggctt gatgttcgcc ctatggtagt agatgcttaa 60
   aaaacacttg ttggagagaa gttaatgttc agagaaggtt ctcgagacga tgcagccctg 120
   gtaaaagaag ggctgtttga taagttagaa attgggattg cttcagatgt gactattcgc 180
   ga 182
<210> 79
<211> 181
<212> ADN
<213> CHLAMYDOPHILA PNEUMONIAE
<400> 79
   ctaattaaag agatgcaggg tctaggactt gatgttcgtc ctatggtcgt agacgcttaa 60
   aaaatgacgt tttggagaaa ataatgttcg gagaaaattc tcgagacatt ggagttcttt 120
   ctaaagaagg actatttgat aaattagaga taggcatagc ttcagatatt acaattcgtg 180
   a 181
<210> 80
<211> 181
<212> ADN
<213> CHLAMYDOPHILA PNEUMONIAE
<400> 80
   ctaattaaag agatgcaggg tctaggactt gatgttcgtc ctatggtcgt agacgcttaa 60
   aaaatgacgt tttggagaaa ataatgttcg gagaaaattc tcgagacatt ggagttcttt 120
   ctaaagaagg actatttgat aaattagaga taggcatagc ttcagatatt acaattcgtg 180
   a 181
<210> 81
<211> 181
<212> ADN
<213> CHLAMYDOPHILA PNEUMONIAE
<400> 81
   ctaattaaag agatgcaggg tctaggactt gatgttcgtc ctatggtcgt agacgcttaa 60
   aaaatgacgt tttggagaaa ataatgttcg gagaaaattc tcgagacatt ggagttcttt 120
   ctaaagaagg actatttgat aaattagaga taggcatagc ttcagatatt acaattcgtg 180
   a 181
<210> 82
<211> 225
<212> ADN
<213> Klebsiella pneumoniae
<400> 82
   ttgttgaaag agattcgttc gctgggtatc aacatcgaac tggaagacga gtaattctcg      60
   ctcaaacagg tcactgctgt cgggttaaaa cccggcagcg gattgtgcta actccgacgg     120
   gagcaaatcc gtgaaagatt tattaaagtt tctgaaagcg cagactaaaa ccgaagagtt     180
   tgatgcgatc aaaattgctc tggcttcgcc agacatgatc cgttc                     225
<210> 83
<211> 225
<212> ADN
<213> ESCHERICHIA COLI
<400> 83
   ttgttgaaag agattcgttc gctgggtatc aacatcgaac tggaagacga gtaattctcg      60
   ctcaaacagg tcactgctgt cgggttaaaa cccggcagcg gattgtgcta actccgacgg     120
   gagcaaatcc gtgaaagatt tattaaagtt tctgaaagcg cagactaaaa ccgaagagtt     180
   tgatgcgatc aaaattgctc tggcttcgcc agacatgatc cgttc                     225
<210> 84
<211> 225
<212> ADN
<213> ESCHERICHIA COLI
<400> 84
   ttgttgaaag agattcgttc gctgggtatc aacatcgaac tggaagacga gtaattctcg      60
   ctcaaacagg tcactgctgt cggggtaaaa cccggcagcg gattgtgcta actccgacgg     120
   gagcaaatcc gtgaaagatt tattaaagtt tctgaaagcg cagactaaaa ccgaagagtt     180
   tgatgcgatc aaaattgctc tggcttcgcc agacatgatc cgttc                     225
<210> 85
<211> 225
<212> ADN
<213> ESCHERICHIA COLI
<400> 85
   ttgttgaaag agattcgttc gctgggtatc aacatcgaac tggaagacga gtaattctcg     60
   ctcaaacagg tcactgctgt cggggtaaaa cccggcagcg gattgtgcta actccgacgg    120
   gagcaaatcc gtgaaagatt tattaaagtt tctgaaagcg cagactaaaa ccgaagagtt    180
   tgatgcgatc aaaattgctc tggcttcgcc agacatgatc cgttc                    225
<210> 86
<211> 225
<212> ADN
<213> Escherichia coli
<400> 86
   ttgttgaaag agattcgttc gctgggtatc aacatcgaac tggaagacga gtaattctcg     60
   ctcaaacagg tcactgctgt cggggtaaaa cccggcagcg gattgtgcta actccgacgg    120
   gagcaaatcc gtgaaagatt tattaaagtt tctgaaagcg cagactaaaa ccgaagagtt    180
   tgatgcgatc aaaattgctc tggcttcgcc agacatgatc cgttc                    225
<210> 87
<211> 225
<212> ADN
<213> SALMONELLA TYPHIMURIUM
<400> 87
   ctgttgaaag agatccgctc gctgggcatc aacatcgaac tggaagacga gtaattctcg     60
   ctcaaacagg tcactggtgt cggggtaaaa cccgacacca gattgtgcta actccgacgg    120
   gagcaaatcc gtgaaagatt tattaaagtt tctgaaagcg cagactaaaa ccgaagagtt    180
   tgatgcgatc aaaattgctc tggcttcgcc agacatgatc cgttc                    225
<210> 88
<211> 225
<212> ADN
<213> Enterobacter cloacae
<400> 88
   ctgttgaaag agattcgttc gctgggtatc aacatcgaac tggaagacga gtaattctcg 60
   ctcaaacagg tcactggtgc cgggttaacc cccggcaccg gattgtgcta actccgacgg 120
   gagcaaatcc gtgaaagatt tattaaagtt tctgaaagcg cagactaaaa ccgaagagtt 180
   tgatgcgatc aaaattgctc tggcttcgcc agacatgatc cgttc 225
<210> 89
<211> 225
<212> ADN
<213> Citrobacter freundii
<400> 89
   ctgttgaaag agattcgttc gctgggtatc aacatcgagc tggaagacga gtaactctcg 60
   atcaaacagg tcactggtgc tggcgtaata gccagcgcca gattgtgcta actccgacgg 120
   gagcaaatcc gtgaaagatt tattaaagtt tctgaaagcg cagactaaaa ccgaagagtt 180
   tgatgcgatc aaaattgcgc tggcctcgcc agacatgatc cgttc 225
<210> 90
<211> 225
<212> ADN
<213> Klebsiella oxytoca
<400> 90
   ttgttgaaag agattcgctc gctgggcatc aacatcgaac tggaagacga gtaactctcg 60
   ctcaaacagg tcactggtgc cggggtaaga cccggcgcca gattgtgcta actccgacgg 120
   gagcaaatcc gtgaaagact tattaaagtt tctgaaagcg caaactaaaa ccgaagagtt 180
   tgatgcgatc aaaattgctc tggcatcgcc agacatgatc cgttc 225
<210> 91
<211> 267
<212> ADN
<213> Serratia liquefaciens
<400> 91
   ctgttgaaag aaatccgctc gctcggtatc aacatcgaac tggaagacga gtaatcgttt    60
   ttccagctca ggctcccggc cttagggagc ctgagggtgg ttgttcaggt cacacgggtg   120
   cgcgatttgt cagcgtgcac ccaacaggtt taactccgac aggagccaat ccgtgaaaga   180
   cttattgaag tttctgaaag cgcaaactaa gaccgaagag tttgatgcga tcaagattgc   240
   tctggcatcg ccagacatga tccgttc                                       267
<210> 92
<211> 267
<212> ADN
<213> Serratia marcescens
<400> 92
   ctgttgaaag aaatccgctc gctcggcatc aacatcgaac tggaagacga gtaatcgtca    60
   tgccggctca ggctccccgc ctaagggagc ctgagggtgg ttgttcaggt cacacgggta   120
   cctactgcgg ttgtgggtac ccaacaggtt taactccgac aggagccaat ccgtgaaaga   180
   cttattgaag tttctgaaag cgcaaactaa gaccgaagag tttgatgcga tcaagattgc   240
   tctggcctcg ccagacatga tccgttc                                       267
<210> 93
<211> 257
<212> ADN
<213> Morganella morganii
<400> 93
   ttgctgaaag aaatccgttc cctcggtatc aatatcgagc tggaagacga gtaattaccg    60
   ttgtggctgc ccgtggtaca cgggcagcac cagtaaatct ggtttaaggg acaaacagac   120
   gaccgtttgt ctcacaggtc taactccgac aggagccatt tcgtgaaaga cttattaaag   180
   tttctgaaag cgcaaaccaa gaccgaagag tttgatgcga tcaaaattgg tctggcctca   240
   cctgacatga ttcgttc                                                  257
<210> 94
<211> 271
<212> ADN
<213> Proteus mirabilis
<400> 94
   ttgttgaaag agatccgttc actgggtatc aacatcgaat tggaagacga ataacgtatt      60
   ccatgaaagc agactgctaa atatggcagt ctgctaaaca gtgactacac tggtttaaag     120
   gggtgaatga caggggtcat ttgcctggca ggtctaactc cgacaggagc catttcgtga     180
   aagacttatt aaagtttctg aaagcgcaaa ccaagaccga agagtttgat gcgatcaaaa     240
   ttgctctggc atcacctgat atgatccgtt c                                    271
<210> 95
<211> 253
<212> ADN
<213> VIBRIO CHOLERAE
<400> 95
   ctgttgaaag agattcgctc gctgggtatc aacatcgagc tagaagacga ataataaacc      60
   ctaaggtttc cccgcaaggg gaagcctacc ggtttcggta ggaaggtgct cgttgccaat     120
   cgcagcgagt tccttttaac tccttacagg agctgaatgt gaaagactta ttaaactttc     180
   taaaagcaca gcataagacc gaagaatttg atgcgatcaa aatcggtctg gcttcaccag     240
   acatgatccg ttc                                                        253
<210> 96
<211> 214
<212> ADN
<213> Pseudomonas aeruginosa
<400> 96
   ctgatcaaag agatccgttc gctcggcatc gacatcgaac tggaaaccga ataacacgtg      60
   acgctagacg gtgcggctgg tcaaggccgg tcgcaccggg tccgtgagga ggaaaggcct     120
   tgaaagactt gcttaatctg ttgaaaaacc agggtcaaat cgaagagttc gatgccatcc     180
   gtattggcct ggcttcgccc gagatgattc gttc                                 214
<210> 97
<211> 214
<212> ADN
<213> PSEUDOMONAS AERUGINOSA
<400> 97
   ctgatcaaag agatccgttc gctcggcatc gacatcgaac tggaaaccga ataacacgtg     60
   acgctagacg gtgcggctgg tcaaggccgg tcgcaccggg tccgtgagga ggaaaggcct    120
   tgaaagactt gcttaatctg ttgaaaaacc agggtcaaat cgaagagttc gatgccatcc    180
   gtattggcct ggcttcgccc gagatgattc gttc                                214
<210> 98
<211> 212
<212> ADN
<213> PSEUDOMONAS PUTIDA
<400> 98
   ttgatcaaag agatccgttc gctcggtatc gatatcgatc tggaaaccga ataacacgtg     60
   acgcgaaggg gagtggggca ggtaatgctg ctccctgctc cgccaggagg aaaggccttg    120
   aaagacctac tgattttgct gaaaaaccag ggtcaagtcg aagagttcga cgccatccgc    180
   atcggtctgg cgtcgcctga aatgatccgt tc                                  212
<210> 99
<211> 228
<212> ADN
<213> SHEWANELLA VIOLACEA
<400> 99
   ttgttgaagg aaatccgttc actcggtatt aatatcgagt tggatcaaga ctaaaattaa     60
   cttaggttaa tttggcaata aattggtgtc ctgcattagc ggggcacccg gtttactcct    120
   tcaggagaga aacgtgaaag acttattaaa gtttctgaaa cagcaaagca agaccgaaga    180
   atttaacggt atcaagatcg gactagcgtc accagatctg atccgctc                 228
<210> 100
<211> 393
<212> ADN
<213> HAEMOPHILUS INFLUENZAE
<400> 100
   attatgaaag aaatccgctc acttggttta aatatcgagt tagacgaaga gtaatcactg     60
   attactataa atggtgctga tcccttggct ccacccgttt acgggggagc tggcgcgaag    120
   actgaggggg gatttatatc ctaagccccc ttccgccctt cgggcacctt ccctcgcaaa    180
   gcaggggaag gcaagaggaa caacaacata agatttgaaa tcgccgaagt gcggtcaaaa    240
   ttctccgaaa tttttaaccg cactttaaac ctttaactcc gacaggagaa catttgtgaa    300
   agacttagtt aagtttttaa aagcacaatc aaaaaccagt gaagattttg atgtgattaa    360
   aattgggtta gcttccccag atatgatccg ttc                                  393
<210> 101
<211> 262
<212> ADN
<213> PASTEURELLA MULTOCIDA
<400> 101
   attatgaaag aaattcgctc gcttggtatc aatattgatt tagatgaaga ttaatctgac     60
   atcataacca agcttggtgt aaagcaatgt acgcgcaagt gcggtaaaaa tttttaaaat    120
   ttcagccgca cttgaataag tttaactccg acaggagcaa atctgtgaaa gacttagtta    180
   agtttttaaa agcacaatca aaaacaagtg aagattttga tgtgatcaaa attggtttag    240
   cctcaccgga catgatccgt tc                                              262
<210> 102
<211> 306
<212> ADN
<213> NEISSERIA MENINGITIDIS
<400> 102
   ttggtcaaag agattcgctc actgggcttg gatatcgatt tggaacgcta ctgatacggg     60
   tttcagacgg cataagggga gctgttctgc aggtatgcgg ggcagccgac aatgtttaaa    120
   aacgaaatgc cgtctgaaaa cactgtacct ctatccatat cgaaaatccg ccatgcggta    180
   aaaatacttc cttcaaggag caaaaatgaa tttgttgaac ttatttaatc cgttgcaaac 240
   tgccggcatg gaagaagagt ttgatgccat caaaatcggt attgcctctc ccgaaaccat 300
   ccgctc 306
<210> 103
<211> 311
<212> ADN
<213> NEISSERIA MENINGITIDIS
<400> 103
   ttggtcaaag agattcgctc actgggcttg gatatcgatt tggaacgtta ctaaacaaaa 60
   gttttcagac ggcctttcag ggtcgtctga aaaagtggtt tcagaataag aatgaagcaa 120
   tcggcattta ggccgtctga aatcaaaagt accgtttccc aatatcgaaa atccgccatg 180
   cggtaaaaat acttccttca aggagcaaaa atgaatttgt tgaacttatt taatccgttg 240
   caaactgccg gcatggaaga agagtttgat gccattaaaa tcggtattgc ctctcccgaa 300
   accatccgct c 311
<210> 104
<211> 226
<212> ADN .
<213> BUCHNERA SP
<400> 104
   cttttaaaag aaattcggtc attagggatt aatattgaac tagaaagcga ataacaaaat 60
   tagcaatatt ataaaaatat ttatgtatta tttatttacc ttaaaagttt tactccaacg 120
   agagctaacg tgtgaaagat ttactaaaat ttctaaaatc ccaaactaaa aatgaagatt 180
   ttgatgctat taaaatctcg ttagcttcac ctgatatgat cagatc 226
<210> 105
<211> 247
<212> ADN
<213> XYLELLA FASTIDIOSA
<400> 105
   ctcgtgaaag aaatccgctc cttagcaatt aatattgagt tggaagataa ctaagatgcg 60
   ttgttatgga ttaattcatc tgtttggagg cccagagctc cattgtcctc tgtttccaac 120
   tcgtcccgat gcccgaattt cggagaagaa gtatgaaaga tctactcaat ctttttaatc 180
   agcagcgcca gacattggat ttcgatgcca tcaagattgg ccttgcctcg cctgccttga 240
   ttagatc 247
<210> 106
<211> 265
<212> ADN
<213> CAULOBACTER CRESCENTUS
<400> 106
   ctggtcaagg aaatgcgctc gctcggcctg aacgtcgagc tggagaacag ctgatctgga 60
   tctccctcct cgcctgcccc tcttaggaag ggtggccggg gaggggcctc ctttcagccc 120
   gctctccctc aagaattttc gcgggaaacc ccgcagaagg aaccaagatg aaccaggaag 180
   tcctgaacat cttcaatccg gtccaggccg ctccgacctt cgaccagatc cgtatctcgc 240
   tcgcctcgcc ggaaaagatc cgctc 265
<210> 107
<211> 325
<212> ADN.
<213> MEZORHIZOBIUM LOTI
<400> 107
   ctcgtcaagg aaatgcggtc tctcggcctc aatgtcgagc tggagaacac caagctcgac 60
   gacgcccctg tccggctgcc cgacgcggcc gagtaaaggc tacagcgcgc cgcacgaagt 120
   tgcggcgcgc aaaggaattc gacggccggt ggccgacaaa agatggcggg cgtttggccc 180
   gcgactagat gcaaggggtt ttcgaggacc ccgaaaagga gaacggcatg aaccaagagg 240
   tcatgaatct cttcaatccg caggcgcctg cgcaggtgtt cgattccatc cggatctcac 300
   tggccagccc tgagaagatt ctgtc 325
<210> 108
<211> 311
<212> ADN
<213> RICKETTSIA PROWASEKII
<400> 108
   atgataaaag aatttagatc tttatgtctc aacgtaaagc ttgaagtaac tccaagtaaa 60
   taaagtgtat atatgttgta cataatttgt cttgttgtat aatttaaaaa ttgttattgc 120
   aagccaaact aaatgaatgt agtgagccat aatgttattt tgtatttaag ctatggagta 180
   acattttaga gtaggagtaa tttttaggga aaagtattta tgagcgtagt taatttttat 240
   ggacaattaa gtaatactca acaatttgac cagataagga ttaatatagc cagtcctgat 300
   caggtacgtt c 311
<210> 109
<211> 188
<212> ADN
<213> BORRELIA BURGDORFERI
<400> 109
   ctaatgcaag agcttagagg gcttggactt gatttgtcaa tttatgatga tgctgggaat 60
   caggttcctt tgacagaaaa agaagaagaa ttgattaata aaagctaggt ttttggagtt 120
   tttatgaaag agataaaaga ttttgaaaga ataaaaatta aaatagcgtc tcccgatcaa 180
   attagaaa 188
<210> 110
<211> 197
<212> ADN
<213> TREPONEMA PALLIDUM
<400> 110
   ttggtgcagg agctgcgggg acttgcgctc gactttacga tttacgatgc gaagggcaag 60
   cagattccgc tcactgagcg cgatgaagaa atgacgaata agattggctc taaattttaa 120
   ggggtgcagg gaatgaagga tatccgggat tttgacagtt tacagataaa gcttgcctcc 180
   cctgatacca ttcgggc 197
<210> 111
<211> 159
<212> ADN
<213> CAMPYLOBACTER JEJUNI
<400> 111
   ttaaccaatg agcttaaatc tcttgcttta gatgttgaga tttttgataa ggatgaagat     60
   aatgagtaaa tttaaagtaa tagaaattaa agaagatgca agacctagag attttgaagc    120
   atttcaacta agacttgcaa gtcctgaaaa aatcaaatc                           159
<210> 112
<211> 161
<212> ADN
<213> HELICOBACTER PYLORI
<400> 112
   ttgactaaag aattgcaatc gctcgctttg gatattaata tttttgggga cgatgtggat     60
   gaggatggag cacctaaacc cattgtcatt aaagaagatg acaggcctaa agactttagc    120
   tctttccagc tcacactagc tagccctgaa aaaatccatt c                        161
<210> 113
<211> 161
<212> ADN
<213> HELICOBACTER PYLORI
<400> 113
   ttgactaaag aattgcagtc gctcgctttg gatattaata tttttgggga cgatgtggat     60
   gaagatggag cgcctagacc cattatgatc aaagaagatg acaggcctaa agactttagc    120
   tctttccagc tcactctagc tagccctgaa aagatccatt c                        161
<210> 114
<211> 175
<212> ADN
<213> AQUIFEX AEOLICUS
<400> 114
   ctcgtaagag agctaaaggc tcttgggcta aacgttaagt gtctgaatgg tgaagagaag 60
   ccttgtgacg aggttgaagt taaagaggag gaagaaaaat gagtgaagca agaaggggta 120
   tcttcccctt ctcaaaaatt aaattgatgc tcgcttctcc cgaggatatc agaag 175
<210> 115
<211> 175
<212> ADN
<213> AQUIFEX PYROPHILUS
<400> 115
   ctcgttaggg agctcaaagg tctcagcctt aacgttaagt gtatgaacgg tgaggagaag 60
   ccctgtgacc aagttgagat taaagaggag gaagaaaaat gagcacaaaa ggtaggggta 120
   tctttccttt ctcaaaaatt aagcttatgc tcgcttctcc cgacgatatc agaag 175
<210> 116
<211> 293
<212> ADN
<213> DEINOCOCCUS RADIODURANS
<400> 116
   ctggtcaagg aactccactc gctcggtctg gacgtcgagg tgctcgacca cggcgacaag 60
   gccgtggaca tctttgaagg gatgatgccc aagcgctaag gcgcctgcgg cactgccaac 120
   ccgtcgagca ctgtcaaacc gtctaaaggt caaaccgcca acatctttca gccgttcgac 180
   ggtgagacag ttcgacggtt tgaccaacaa aagagcctcc attccacagg agcctgaatg 240
   aaagacttca acaaagtccg catcgccatc gccagcccgg agaagatccg cga 293
<210> 117
<211> 177
<212> ADN
<213> THERMOS AQUATICUS
<400> 117
   ctggtgaagg agcttcaggc cctggccctg gacgtgcaga ccctggacga gaaggacaac 60
   cccgtggaca tttttgaggg cctggcctcc aagaggtgag cccttttctg gaggaaagat 120
   gaaaaaggaa gtccgcaagg tccgcatcgc cctggcctcc cccgagaaga tccgctc 177
<210> 118
<211> 174
<212> ADN
<213> THERMOTOGA MARITIMA
<400> 118
   ctcatcaaag aactcagagg tctcgcgctc gatgtgagac tctacgatga gaacggtaac 60
   gagatagata tcgacaagta ctgattggga ggttggtaga atgccaatgt cctctttcaa 120
   gaggaagata aaggcaattc agataaagat agcctctccg gaagtgataa gaag 174
<210> 119
<211> 324
<212> ADN
<213> STREPTOMYCES COELICOLOR
<400> 119
   ctcatcaagg agatgcagtc cctgtgcctc aacgtggagg tgctgtcctc ggacggcatg 60
   tccatcgaga tgcgtgacac cgacgaggac gtcttccgcg cagcggagga gctcggcatc 120
   gacctgtcgc ggcgcgagcc gagcagcgtc gaagaggtct gacgggagtc aggcggggcc 180
   tgtcctccac aggccccgcc gatcccgcga cccccgtttc agaccacaga cttacaaccc 240
   tgagagggat tgacgcatag tgctcgacgt caacttcttc gacgagctcc ggatcggtct 300
   ggccaccgct gacgacatcc gtca 324
<210> 120
<211> 281
<212> ADN
<213> MYCOBACTERIUM LEPRAE
<400> 120
   ctgctcaagg agttacagtc gctgtgtctc aacgtcgagg tgctgtcgtc cgacggtgcg 60
   gcgatcgagt tgcgcgaagg tgaggatgag gacctcgagc gggctgcggc caacctcggt 120
   atcaacttgt cccgcaacga atcggcgtcc atagaagatc tggcttagcg aacttggcat 180
   tatcgtcact aaacccgcaa ggggaaaggg agttacgtgc tagacgtcaa cttcttcgat 240
   gaactccgca ttggcctggc taccgcggag gacattcgtc a 281
<210> 121
<211> 277
<212> ADN
<213> MYCOBACTERIUM TUBERCULOSIS
<400> 121
   ctgctcaaag aactgcagtc gctgtgcctc aacgtcgagg tgctatcgag tgacggtgcg 60
   gcgatcgaac tgcgcgaagg tgaggacgag gacctggagc gggccgcggc caacctggga 120
   atcaatctgt cccgcaacga atccgcaagt gtcgaggatc ttgcgtaaag ctgtcgcaaa 180
   attactaaac ccgttagggg aaagggagtt acgtgctcga cgtcaacttc ttcgatgaac 240
   tccgcatcgg tcttgctacc gcggaggaca tcaggca 277
<210> 122
<211> 277
<212> ADN
<213> MYCOBACTERIUM TUBERCULOSIS
<400> 122
   ctgctcaaag aactgcagtc gctgtgcctc aacgtcgagg tgctatcgag tgacggtgcg 60
   gcgatcgaac tgcgcgaagg tgaggacgag gacctggagc gggccgcggc caacctggga 120
   atcaatctgt cccgcaacga atccgcaagt gtcgaggatc ttgcgtaaag ctgtcgcaaa 180
   attactaaac ccgttagggg aaagggagtt acgtgctcga cgtcaacttc ttcgatgaac 240
   tccgcatcgg tcttgctacc gcggaggaca tcaggca 277
<210> 123
<211> 192
<212> ADN
<213> PORPHYROMONAS CANGINGIVALIS
<400> 123
   ctcctacacg agctcaaagg tcttggtcta agcttctgta tggagtaata ggcgaggata 60
   tgtgattata gttttttcct catcagaata aatctcccat tatatagtta tggcattcaa 120
   aagagataca aagataaagg ccaacttcac ccgtattaag atcggtatcg cttctcccga 180
   agaggtattg ga 192
<210> 124
<211> 257
<212> ADN
<213> MYCOPLASMA GENITALIUM
<400> 124
   ttgacaaaag aattacaggg cttggcttta tctgtttcat ttatctatga tgacaacacc 60
   caacaagact ccaataatgt ttccatcttg caaagtgatg gggaacaaga tgaatttttc 120
   aatgattttg aatttgacac tgagggttat tagaaattaa caatgacaac aacaagacgt 180
   aataaaagaa ataacaagct ttataaaaac attaaagcaa ttaaactttc catcgcttcc 240
   aatgacacca ttttgaa 257
<210> 125
<211> 245
<212> ADN
<213> MYCOPLASMA PNEUMONIAE
<400> 125
   ttaacgaagg aactacaagg gttagcgttg agtgtgtcct ttatttacga tgacaacacc 60
   caacaagatt ccaacaacgt ttcaattctc caagctgatg gagaacagga cgatctcttt 120
   aatgactttg aatttgacac ggagggttat taattaatga caaagcgtaa taaaaagaac 180
   aacaagctgt acaagaacat taaggcaatt aagctttcga ttgcttccaa cgacacgatc 240
   ctaaa 245
<210> 126
<211> 305
<212> ADN
<213> UREAPLASMA UREALYTICUM
<400> 126
   ttaacaaaac aaatgcaagg tttagggtta tgtattaccg ttgaaacaaa agatgatcgt     60
   atggtagata ttaatgaata tacactaaat caaaatcgtt taaataatga cgatgatgag    120
   gttattttag atgaaaatct aaaagagatc aatgattcta atgaagaaat atttaataca    180
   aactttaata ataatgacta tgatgatgaa gagaacttct aaataataga aaggtaaaat    240
   aatatgagtc aaaaagggat taaatcatta acgatttcca ttgcttcacc tgaacaaatt    300
   ttaaa                                                                305
<210> 127
<211> 244
<212> ADN
<213> MYCOPLASMA PULMONIS
<400> 127
   ttagcctatg aattaagagg gctaggaatc aaacttcaaa ttcatgaaaa agaagaagaa     60
   aaacaagaac taccaagcca agaatatgaa agtttaaatc ttgatcaaga gctaaaaaca    120
   gcttctgaaa atgttagtga aagtgagttt taattatgcc aaaaactaga aaatattcaa    180
   cagttgatga agaaaagatt ttaaaagtta gcttatctct tgcaactaaa gaagatgttt    240
   taga                                                                 244
<210> 128
<211> 202
<212> ADN
<213> PLASMODIUM FALCIPARUM
<400> 128
   attttaaaag agttacaaag tttagctatt aatatagaag ctttttgtat atttaatgat 60
   acaaataatt tattagaaaa tttacctatt aatataattt attaataatg ataatacata 120
   ataatataaa ttttatagga ttaaaattaa atatattaaa tcctaaacaa ataataaaat 180
   ggtcttcaat attttataaa aa 202
<210> 129
<211> 136
<212> ADN
<213> ARCHAEOGLOBUS FULGIDUS
<400> 129
   cttctggatg agctgaagtc aatgatgatc gctccgagaa taattctcgg agataaggca 60
   tgaggtgaaa tgagatggta ccgaagagga tttcagccat taaatttgag gttctctccc 120
   cccaagagat aagaag 136
<210> 130
<211> 169
<212> ADN
<213> METHANOTHERMOBACTER THERMOAUTOTROPHICUS
<400> 130
   ttacttctcg aactcaagag tctctgtatc ttcccgaaac tcatactgga agataaggca 60
   tgataatgga tttaagggaa taacaaaaag gagagaatac cttgagagga attttaaaga 120
   aaatttccca gataaacttt ggcctcatgt cccccgagga tatcaggaa 169
<210> 131
<211> 136
<212> ADN
<213> HALOBACTERIUM SP
<400> 131
   ctactcgacg agatgaaggc gctcggcatc gcgccgcgcc tggaactgga ggaggcagtg 60
   taatgagtgc aggacaagcc cccaaggaaa tcggcgaaat cagcttcggg ctgatggacc 120
   cagaggagta ccgcga 136
<210> 132
<211> 127
<212> ADN
<213> THERMOPLASMA VOLCANIUM
<400> 132
   atgagggatg agctgatatc tctcggtgtt gttatgcgtc ttatgttggg tgatatgaaa 60
   tgatgggaat ttctaaaaga atttcaagta ttaaatttgc gcttctttct ccagacgaga 120
   taagaaa 127
<210> 133
<211> 127
<212> ADN
<213> THERMOPLASMA ACIDOPHILUM
<400> 133
   atgagggatg agctgatctc tctcggtgtt gttatgaggt taatgctcgg tgatatgaaa 60
   tgatgggaat atcaaaaaga atttcatcaa taaaatttgc ccttctttct ccggatgaga 120
   taagaaa 127
<210> 134
<211> 141
<212> ADN
<213> SULFOLOBUS ACIDOCALDARIUS
<400> 134
   ttaattcaag aacttatgag tatggtaatt tcaccgagat taattttagg tgaaaaagta 60
   aacttaggag gtgcttcaaa tgagtgagaa gattatacgg ggcgtaaaat ttggtgtatt 120
   atcacctaat gaaataaggc a 141
<210> 135
<211> 145
<212> ADN
<213> SULFOLOBUS SOLFATARICUS
<400> 135
   ttaattcaag aactaatgag tatgattatc tcacctaggt tagttttgga ggataaagtt 60
   ggattaagtg gaggttaagg gaaatgagtg aaaagaatat aaaaggaata aagtttggaa 120
   tactttctcc tgacgaaata agaaa 145
<210> 136
<211> 134
<212> ADN
<213> PYROCOCCUS ABYSSI
<400> 136
   ctcttggatg agcttaaggc catggttatt aggccaaagt taaacctcac ggagagggtg 60
   tgagctatgc aatccgttaa gaaggttatc ggtagtatag agtttggaat tctctcccct 120
   caagaaatta gaaa 134
<210> 137
<211> 134
<212> ADN
<213> PYROCOCCUS HORIKOSHII
<400> 137
   cttctggatg agcttaaggc tatggtgatt agacctaagt taaacctcac ggagagggtg 60
   tgagccatgc actcagttaa gaaggttata ggtagtattg aatttggaat actttcccct 120
   caagaaatta ggaa 134
<210> 138
<211> 224
<212> ADN
<213> AEROPYRUM PERNIX
<400> 138
   ctgctgcagg agataaccag tatgatgata aagccggaac tcaaggtagc cgacaagata 60
   tccgtcatca gaaaagtcgt cggcgactat acatgattac cccattttaa ttctcggatt 120
   tcgggggtgt tgggtgctat gtctctaagg ctctcggagt tccgcgagac aaaccttcta 180
   gataagatac tctttggcgt cttaagcccc catgagataa ggca 224
<210> 139
<211> 26
<212> ADN
<213> Sequence artificielle
<220>
   <223> Amorce
<220>
   <223> n signifie a,g,c ou t/u
<400> 139
   marccnntng gngaymgngt natngt 26
<210> 140
<211> 186
<212> ADN
<213> PASTEURELLA MULTOCIDA
<400> 140
   gaaaaaattg atggcgaaga agtgttaatt atttctgaaa acgatatttt agccattgtt 60
   gaataatttt tatcaacaac acaaaatcgt tatttctata aataaacaaa cttaaaatag 120
   caatttgcat aacaagattc gaaatgagag gaagataaaa aatggcagca aaagacgtaa 180
   aatttg 186
<210> 141
<211> 113
<212> ADN
<213> HAEMOPHILUS INFLUENZAE
<400> 141
   gaaaaaatcg atggtgaaga agtgttaatc atttctgaaa acgacatcct agcaattgta 60
   gaataattat taaataaggg aaaagaaaat ggcagcaaaa gacgtaaaat ttg 113
<210> 142
<211> 113
<212> ADN
<213> HAEMOPHILUS DUCREYI
<400> 142
   gaaaaaattg atggcgaaga aattttaatt ctttcagaga atgacattct tgcaattgta 60
   gaataatcga agaataaggg ataataaaat ggcaataaaa gacgttaaat ttg 113
<210> 143
<211> 137
<212> ADN
<213> BUCHNERA APHIDICOLA
<400> 143
   gaaaaaattg ataacgaaga attattaatt ctaactgaaa gcgacatttt agcaattgtt 60
   gaatagtaaa ccacatgcta tatcattgaa aattgattta aggggatgtc aaatggccgc 120
   taaagatgta aaatttg 137
<210> 144
<211> 139
<212> ADN
<213> MYZUS PERSICA
<400> 144
   gaaaaaatta atactgaaga gttattactt ttaactgaaa gtgacatttt agcaattgtt 60
   gaatagtaaa ctatatgcta tatccattta aaaatttatt taagggaatg tcaaatggcc 120
   gctaaagatg taaaatttg 139
<210> 145
<211> 144
<212> ADN
<213> VIBRIO CHOLERAE
<400> 145
   gaaaagatcg atggcaaaga agtgctgatc ttggctgaac atgacatttt ggcaatcgtt 60
   gaataattga ttctgaatcc caacgaaatc aataactgaa tttagaaagg aaatgaaaaa 120
   tggctgctaa agacgtacgt tttg 144
<210> 146
<211> 137
<212> ADN
<213> ESCHERICHIA COLI
<400> 146
   gagaagatcg acaatgaaga agtgttgatc atgtccgaaa gcgacattct ggcaattgtt 60
   gaagcgtaat cctcgcacga cactgaacat acgaatttaa ggaataaaga taatggcagc 120
   taaagacgta aaattcg 137
<210> 147
<211> 137
<212> ADN
<213> ESCHERICHIA COLI
<400> 147
   gagaagatcg acaatgaaga agtgttgatc atgtccgaaa gcgacattct ggcaattgtt 60
   gaagcgtaat cctcgcacga cactgaacat acgaatttaa ggaataaaga taatggcagc 120
   taaagacgta aaattcg 137
<210> 148
<211> 137
<212> ADN
<213> ESCHERICHIA COLI
<400> 148
   gagaagatcg acaatgaaga agtgttgatc atgtccgaaa gcgacattct ggcaattgtt 60
   gaagcgtaat ccgcgcacga cactgaacat acgaatttaa ggaataaaga taatggcagc 120
   taaagacgta aaattcg 137
<210> 149
<211> 142
<212> ADN
<213> PSEUDOMONAS PUTIDA
<400> 149
   gtgaaagtcg atggcgaaga cctgctggta atggccgaga acgagattct cgccgttatc 60
   gaaggctgat ttccccgact tcccgttatt ccaaagcatt tcaaggatta aacgatcatg 120
   gctgctaaag acgtaaaatt cg 142
<210> 150
<211> 144
<212> ADN
<213> PSEUDOMONAS AERUGINOSA
<400> 150
   atcaaggtcg atggcgagga actgctggtg atgggcgagt ccgaaatcct cgccgtcctg 60
   gaagactgat cggtctcacc actccgtttt caccgaattc gatttagagg aaagagaaca 120
   tggctgccaa agaagttaag ttcg 144
<210> 151
<211> 186
<212> ADN
<213> NEISSERIA MENINGITIDIS
<400> 151
   gtaaaagccg acggcgaaga gctgttggta atgcgcgaag aagatatttt cggcatcgtt     60
   gaaaaataaa tacggacacg atgccgtctg aaacggcaaa ccgccttcag acggcataaa    120
   cggttttatc agacagtttt aatgattttt ggagaattga aatggcagca aaagacgtac    180
   aattcg                                                               186
<210> 152
<211> 186
<212> ADN
<213> NEISSERIA MENINGITIDIS
<400> 152
   gtaaaagccg acggcgaaga gctgttggta atgcgcgaag aagatatttt cggcatcgtt     60
   gaaaaataaa tacggacacg atgccgtctg aaacggcaaa ccgccttcag acggcataaa    120
   cggttttatc agacagtttt aatgattttt ggagaattga aatggcagca aaagacgtac    180
   agttcg                                                               186
<210> 153
<211> 185
<212> ADN
<213> Neisseria gonorrhoeae
<400> 153
   gtaaaagccg acggcgaaga gctgttggta atgcgcgaag aagatatttt cggcatcgtt     60
   gaaaaataaa tacggacacg atgccgtctg aaacggcaaa ccgccttcag acggcataaa    120
   cggttttatc agacagtttt aagattttgg gagaattgaa atggcagcaa aagacgtaca    180
   attcg                                                                185
<210> 154
<211> 201
<212> ADN
<213> XYLELLA FASTIDIOSA
<400> 154
   tacaaggctg aaggcgtcga atacaaagta ttacgcgagg acgacatcct ggcgatcatc    60
   ggttgattaa gccaagcccg aaactcgtga atgcatccga catatcacgc caacagcggg   120
   cacattgttc catacatcac taatgttctc atcgcgaatc ttggagtaaa aacataatgg   180
   ctgccaaaga aattattttc a                                             201
<210> 155
<211> 224
<212> ADN
<213> STREPTOMYCES COELICOLOR
<400> 155
   gtgaagtaca acggcgagga gtacctcgtc ctctcggccc gcgacgtgct cgcgatcgtc    60
   gagaagtaga agtagtactt cgcttcaccg aagcaccttg ctttccagct gcgcccctgg   120
   ctcccgcgac cataaaaagc cgggcgtcgg gggcgcagtt gccgtataac cccaagattt   180
   ccggaagagg gctcacgctc ccatggcgaa gatcctgaag ttcg                    224
<210> 156
<211> 185
<212> ADN
<213> MYCOBACTERIUM TUBERCULOSIS
<400> 156
   atcaagtaca acggcgagga atacctgatc ctgtcggcac gcgacgtgct ggccgtcgtt    60
   tccaagtagt agagcgtgtt ccgccccggc gatccccgtg ctcaccacgg gtgatttccg   120
   gggcggcatg cgttagcgga ctagccctgc gtagaggagc ctgatgagca agctgatcga   180
   atacg                                                               185
<210> 157
<211> 185
<212> ADN
<213> MYCOBACTERIUM TUBERCULOSIS
<400> 157
   atcaagtaca acggcgagga atacctgatc ctgtcggcac gcgacgtgct ggccgtcgtt     60
   tccaagtagt agagcgtgtt ccgccccggc gatccccgtg ctcaccacgg gtgatttccg    120
   gggcggcatg cgttagcgga ctagccctgc gtagaggagc ctgatgagca agctgatcga    180
   atacg                                                                185
<210> 158
<211> 169
<212> ADN
<213> MYCOBACTERIUM LEPRAE
<400> 158
   atcaagtaca atggcgagga atacctgatc ctgtcggcac gtgacgtgct ggctgtcgta     60
   tccaagtaac gaaccgtgtt ccgccccggc gatccccgtg cttaaccacg ggtgatttcc    120
   ggggcggcat gcgtttaaag gagcctgatg agcaagctga ttgagtacg               169
<210> 159
<211> 103
<212> ADN
<213> THERMUS AQUATICUS
<400> 159
   attgagattg caccgcgaag gacgtacgtg atcctctccg agcgcgacct gcttgcggtc     60
   ctgcagtaaa ggaggtgaac catggcgaag atcctggtgt ttg                     103
<210> 160
<211> 100
<212> ADN
<213> THERMOS THERMOPHILUS
<400> 160
   attgagattg acggcgagga gtacgtgatc ctctccgagc gcgacctgct tgcggtcctg 60
   cagtaaagga ggtgaaccat ggcgaagatc ctggtgtttg 100
<210> 161
<211> 100
<212> ADN
<213> THERMUS THERMOPHILUS
<400> 161
   attgagattg acggcgagga gtacgtgatc ctctccgagc gcgacctgct tgcggtcctg 60
   cagtaaagga ggtgaactat ggcgaagatc ctggtgtttg 100
<210> 162
<211> 162
<212> ADN
<213> DEINOCOCCUS RADIODURANS
<400> 162
   gtcagcctcg aaggcaagaa ctacagcctg ctgagcgagc gcgacctgct cgccattgtc 60
   gagtaaggct ccgagtcagg ttctgagcct gttcgtttcc tgtttttctt cctcatttca 120
   cttttcaagg agcaatcaca atggctaaac agctcgtgtt tg 162
<210> 163
<211> 121
<212> ADN
<213> PORPHYROMONAS GINGIVALIS
<400> 163
   atagagctgg agggcgaaaa atatatcatc atgcgccaaa acgatgtctt ggcaatcatc 60
   taattctcag agacaataac ctacaataaa aaataaagac tatggcaaaa gaaatcaaat 120
   t 121
<210> 164
<211> 134
<212> ADN
<213> BACILLUS SUBTILIS
<400> 164
   gtgaaatacg aaggtactga atacttaatc ttacgtgaaa gcgacatttt agctgttatc     60
   ggctaattct taaataaaca atacttaaaa catttgagga ggtcttgtaa acatggcaaa    120
   agaaattaag ttta                                                      134
<210> 165
<211> 180
<212> ADN
<213> BACILLUS HALODURANS
<400> 165
   gtaaaatatg atggtaaaga gtatttaatc cttcgtgaaa gcgatattct cgcgattatc     60
   ggttaatttt acgtagggtt atccctacat acatgtaaga cgagaggttt ttgtctattc    120
   ctcttttgta aaataccatt caggaggttg agaataacat ggcaaaagat attaagttta    180
<210> 166
<211> 121
<212> ADN
<213> LACTOBACILLOS ZEAE
<400> 166
   gtgaagtatg aaggtcaaga ctaccttgta ttgcatgaaa aagacatcat ggcaattgcg     60
   taactaaata atcgatcaat tttgaggtga ataaaaacaa tggcaaaaga aattaaattc    120
   t                                                                    121
<210> 167
<211> 142
<212> ADN
<213> Clostridium perfringens
<400> 167
   gttaagttcg agggggaaga atatactatt ttaagacaag acgatatact agcaatagtt 60
   gaatagtttt aaaatataag tgatttagat attcataata tatttgggag gtaaattaat 120
   atggctaaaa cattattatt cg 142
<210> 168
<211> 120
<212> ADN
<213> CLOSTRIDIUM DIFFICILE
<400> 168
   gttaagatag aaggacaaga atacacaata ctaagacaga gtgatgtatt agctgttatt 60
   gaataaatat agaataaatt tattaggagg ggtttaaaat ggctaaagaa attaaatttt 120
<210> 169
<211> 129
<212> ADN
<213> CLOSTRIDIUM ACETOBUTYLICUM
<400> 169
   ataaaagttg acaatgaaga attgttaatt ttaagacagg acgatatttt aggaattgta 60
   gaagaataag ctatcaattt tgttaataat tcagggaggg attctaaatg gcaaagcaaa 120
   tattatacg 129
<210> 170
<211> 141
<212> ADN
<213> LACTOBACILLUS HELVETICUS
<900> 170
<210> 171
<211> 118
<212> ADN
<213> LACTOBACILLUS JOHNSONII
<400> 171
   ttgaagtacg aaggcgaaaa gtacttagtt cttcgtgaaa gcgacttatt agctgtcgtt 60
   aagtaataaa atttgaaata aaaggtggca tataatatgg ctaaagagat taaatttt 118
<210> 172
<211> 143
<212> ADN
<213> STAPHYLOCOCCUS EPIDERMIS
<400> 172
   gtaaaacgtg gcgcccaaac atatttaatt ttaaatgaag aagatatatt agctattata 60
   gaataaagag cgaattttaa atattaatta aatgatttaa taagtggagg ttgtttagac 120
   tatggcaaaa gatcttaaat tct 143
<210> 173
<211> 163
<212> ADN
<213> STAPHYLOCOCCUS AUREUS
<400> 173
   gttaaacgag ataatgaaac atatctagta ttaaatgaag aagatatttt agcggtaatt 60
   gaataatata aaattaaatt catagataaa ttgtaaagaa cgaaaatgaa atatgactaa 120
   acaaatggag gtttatcatt tatggttaaa caattgaaat tct 163
<210> 174
<211> 106
<212> ADN
<213> STREPTOCOCCUS PNEUMONIAE
<400> 174
   gtcaaagatg gcgatgaaaa gtacatcatc gtaggcgaag ctaacatttt ggcaatcatt 60
   gaggaataga aggagaaagt aagtatgtca aaagaaatta aatttt 106
<210> 175
<211> 175
<212> ADN
<213> LACTOCOCCUS LACTIS
<400> 175
   gtaaaaatgg atggtgaaga attcttgatt ctcaaagatt cagaccttct tgcaattgta 60
   gagtaaaatt ataaaagcaa tcattttttt ggttgtcttt tgtctatctt aaaatctata 120
   aaattaaaaa tatattctta aaaaggagct aaaatgtcaa aagatattaa atttt 175
<210> 176
<211> 111
<212> ADN
<213> RICKETTSIA PROWASEKII
<400> 176
   attgaaataa aaggagaaaa attaatcgtt atgaaagaaa gcgatgtatt tggtattatt 60
   aattaattat ttttaggaga aaaaatatga caacgaaact tattaaacac g 111
<210> 177
<211> 129
<212> ADN
<213> CHLAMYDIA MURIDARUM
<400> 177
   ctcactgtcg aaggtgaaga atatgtcatc gttcaaatga gcgaagttat agcagtcctg 60
   caataaaaac taagagagtg aagtaagatt taaggagcgc atcgatggtc gctaaaaata 120
   ttaaatata 129
<210> 178
<211> 128
<212> ADN
<213> CHLAMYDIA TRACHOMATIS
<400> 178
   cttactgtcg aaggtgaaga gtacgtcatc gttcaaatga gcgaagttat cgcagttctg 60
   caataaaaac taagagagtg aagaagattt aaggagcgca tcaatggtcg ctaaaaacat 120
   taaataca 128
<210> 179
<211> 132
<212> ADN
<213> CHLAMYDOPHILA PNEUMONIAE
<400> 179
   atcacaatcg atgacgaaga gtatgtcatt ctacagtcca gtgaaatcat ggccgtccta 60
   aaataaaata ctagtttgca gattatagaa agttaaggag aacaacgatg gcagcgaaaa 120
   atattaaata ta 132
<210> 180
<211> 132
<212> ADN
<213> CHLAMYDOPHILA PNEUMONIAE
<400> 180
   atcacaatcg atgacgaaga gtatgtcatt ctacagtcca gtgaaatcat ggccgtccta 60
   aaataaaata ctagtttgca gattatagaa agttaaggag aacaacgatg gcagcgaaaa 120
   atattaaata ta 132
<210> 181
<211> 132
<212> ADN
<213> CHLAMYDOPHILA PNEUMONIAE
<400> 181
   atcacaatcg atgacgaaga gtatgtcatt ctacagtcca gtgaaatcat ggccgtccta 60
   aaataaaata ctagtttgca gattatagaa agttaaggag aacaacgatg gcagcgaaaa 120
   atattaaata ta 132
<210> 182
<211> 141
<212> ADN
<213> CHLAMYDOPHILA CAVIAE
<400> 182
   cttaccgttg atggtgagga gtacgtcatt gttcaggaaa gcgaagttat ggcagttctc 60
   aagtaagaga aatcattatt tatagattgc aaaaagttaa ggagcacaaa aaaacaatgg 120
   cagcaaaaaa tattaaatat a 141
<210> 183
<211> 160
<212> ADN
<213> HELICOBACTER PYLORI
<400> 183
   ctagaagaca ttctaggcat tgtgggctca ggctcttgtt gtcatacagg taatcatgac 60
   cataaacatg ctaaagagca tgaagcttgc tgtcatgatc acaaaaaaca ctaaaaacat 120
   tattattaag gatacaaaat ggcaaaagaa atcaaatttt 160
<210> 184
<211> 160
<212> ADN
<213> HELICOBACTER PYLORI
<400> 184
   ctagaagaca ttctaggtat tgtgggctca ggctcttgct gtcatacagg taatcatgac 60
   cataaacatg ctaaagagca tgaagcttgc tgtcatgatc acaaaaaaca ctaaaaacat 120
   tattattaag gatacaaaat ggcaaaagaa atcaaatttt 160
<210> 185
<211> 72
<212> ADN
<213> CAMPYLOBACTER JEJUNI
<400> 185
   ttagatgata tcttaggaat tttaaaataa tttataaaaa aggataaaaa atggcaaaag 60
   aaattatttt tt 72
<210> 186
<211> 136
< 212 > ADN
<213> CLOSTRIDIUM THERMOCELLUM
<400> 186
   gtaaaatttg acggacagga atatacgatc ttaagacaaa acgatatttt ggcggtagta 60
   gagtaattat attaccaact tcaatacaaa aagtatccta aggaggttaa tcatatggca 120
   aagcaaataa aatttg 136
<210> 187
<211> 127
<212> ADN
<213> MYCOPLASMA GENITALIUM
<400> 187
   tttgagaatg agggaaacaa gtacaaaatt attggatttg aggatgtact tgcctttgaa 60
   aaaccagaaa gtggtaagca aagaaaaaga taaaattaaa caattatggc aaaggaatta 120
   atctttg 127
<210> 188
<211> 138
<212> ADN
<213> MYCOPLASMA PNEUMONIAE .
<400> 188
   tttgaagagg aaggtaccaa gtacaagatt atttccttgg aagatgtcct tgcttttgaa     60
   aagcatggta atacaaaaac tactactgta aagaaaggag ctaagaaaaa atagttatgg    120
   caaaggaatt agtatttg                                                  138
<210> 189
<211> 120
<212> ADN
<213> AQUIFEX AEOLICUS
<400> 189
   gtagagattg aaggaaagat ttacctcgtt atgtctgaag acgaagtttt agctgttgtt     60
   gaagattatt caagcttaat aggaggtgag gtgagatggc agcaaaggca attatctaca    120